# EUROPEAN PATENT SPECIFICATION

(11) **EP 2 239 335 B1**
(45) Date of publication and mention of the grant of the patent: **27.02.2013**
(21) Application number: 09701789.1
(22) Date of filing: 16.01.2009
(51) Int. Cl.: C12P 13/04, C12P 7/00, C12N 9/10

(54) **PROCESS FOR PRODUCTION OF SERINE DERIVATIVE, AND PROTEIN FOR USE IN THE PROCESS**
HERSTELLUNGSVERFAHREN FÜR SERINDERIVAT UND PROTEIN ZUR VERWENDUNG IN DEM VERFAHREN
PROCÉDÉ DE PRODUCTION D'UN DÉRIVÉ DE SÉRINE, ET PROTÉINE UTILISÉE DANS LEDIT PROCÉDÉ

(30) Priority: 18.01.2008 JP 2008009853
(43) Date of publication of application: 13.10.2010
(73) Proprietor: Ajinomoto Co., Inc., Tokyo 104-8315 (JP)
(72) Inventor: KURODA, Shinji, Kawasaki-shi Kanagawa 210-8681 (JP); SUGIYAMA, Masakazu, Kawasaki-shi Kanagawa 210-8681 (JP); WATANABE, Kunihiko, Kawasaki-shi Kanagawa 210-8681 (JP); SUZUKI, Shunichi, Kawasaki-shi Kanagawa 210-8681 (JP); YOKOZEKI, Kenzo, Kawasaki-shi Kanagawa 210-8681 (JP); KASHIWAGI, Tatsuki, Kawasaki-shi Kanagawa 210-8681 (JP)
(74) Representative: HOFFMANN EITLE
(86) International application number: PCT/JP2009/050575
(87) International publication number: WO 2009/091045

(56) References cited:
- WO-A1-2006/123745
- JP-A- 3 206 892
- JP-A- 2006 320 294
- SCHIRCH V ET AL: "Serine hydroxymethyltransferase revisited", CURRENT OPINION IN CHEMICAL BIOLOGY, CURRENT BIOLOGY LTD, LONDON, GB, vol. 9, no. 5, 1 October 2005 (2005-10-01) , pages 482-487, XP025370014, ISSN: 1367-5931 [retrieved on 2005-10-01]
- NOZAKI HIROYUKI ET AL: "Cloning of the Gene Encoding alpha-Methylserine Hydroxymethyltransferase from Aminobacter sp AJ110403 and Ensifer sp AJ110404 and Characterization of the Recombinant Enzyme", BIOSCIENCE BIOTECHNOLOGY AND BIOCHEMISTRY, vol. 72, no. 11, November 2008 (2008-11), pages 3002-3005, XP002638682, ISSN: 0916-8451

### [Sequence Listing]

### SEQUENCE LISTING

<110> Ajinomoto Co., Inc.
<120> Method for Producing Serine Derivertive and Protein Therefor
<130> PAMA-201034
   (D-003)
<150> JP2008-009853
   <151> 2008-01-18
<160> 15
<170> PatentIn version 3.4
<210> 1
   <211> 23
   <212> PRT
   <213> Rhodococcus sp.
<220>
   <223> Inventor: shinji KURODA
   Inventor: masakazu SUGIYAMA
   Inventor: kunihiko WATANABE
   Inventor: shunichi SUZUKI
   Inventor: kenzo YOKOZEKI
   Inventor: tatsuki KASHIWAGI
<400> 1
   Pro Gly Phe Ser Gln Val Gln Trp Ser Ser Pro Cys Arg Leu Asp Phe
   1               5                   10                  15
   Arg Asn Glu Gly Arg Val Ala
               20
<210> 2
   <211> 24
   <212> DNA
   <213> Artificial
<220>
   <223> primer (SEQA)
<220>
   <221> misc_feature
   <222> (18)..(18)
   <223> n is a, c, g, or t
<400> 2
   ccdgghttyw shcargtnca rtgg 24
<210> 3
   <211> 29
   <212> DNA
   <213> Artificial
<220>
   <223> primer (SEQB)
<220>
   <221> misc_feature
   <222> (15).. (15)
   <223> n is a, c, g, or t
<400> 3
   gghttywshc argtncartg gwshwshcc 29
<210> 4
   <211> 1098
   <212> DNA
   <213> Rhodococcus sp.
<220>
   <221> CDS
   <222> (1)..(1098)
<400> 4
   atg cca ggc ttt tcg cag gtc cag tgg agt tcg ccg ccc cgg ctc gac       48
   Met Pro Gly Phe Ser Gln Val Gln Trp Ser Ser Pro Pro Arg Leu Asp
   1               5                   10                  15
   ttc cgg aac gag ggg cga gtc gcg ccg acc gag gag atg tgg gag gcc       96
   Phe Arg Asn Glu Gly Arg Val Ala Pro Thr Glu Glu Met Trp Glu Ala
               20                  25                  30
   atg cgt cgg gca tcc gag aac ttc gag atg tcc tcc ttc ggg acc gat      144
   Met Arg Arg Ala Ser Glu Asn Phe Glu Met Ser Ser Phe Gly Thr Asp
           35                  40                  45
   ccg agc gtg cgc gaa ctg gaa ctt att ggc gcc gag atg aca ggt cag      192
   Pro Ser Val Arg Glu Leu Glu Leu Ile Gly Ala Glu Met Thr Gly Gln
       50                  55                  60
   gaa gcc tca tac ttc ttg ccg tcg aca acc gcg gcg acg ttg gtg gcg      240
   Glu Ala Ser Tyr Phe Leu Pro Ser Thr Thr Ala Ala Thr Leu Val Ala
   65                  70                  75                  80
   cta ttg gcg gag gac gtg cgc gca aag caa ctc atc gtc gag gcc agg      288
   Leu Leu Ala Glu Asp Val Arg Ala Lys Gln Leu Ile Val Glu Ala Arg
                   85                  90                  95
   tcg cat ctt tac tgg cta cag cgg ttt cac gac tcg gtg cat gcc ggc      336
   Ser His Leu Tyr Trp Leu Gln Arg Phe His Asp Ser Val His Ala Gly
               100                 105                 110
   ggg cgc gcc gcc acg atc gag ggc gac aag ttc ggc gtt atg tcg ctg      384
   Gly Arg Ala Ala Thr Ile Glu Gly Asp Lys Phe Gly Val Met Ser Leu
           115                 120                 125
   agt gac atc gac gag ttg atc aac cgg acc gtt tgg ggc ctt gag aac      432
   Ser Asp Ile Asp Glu Leu Ile Asn Arg Thr Val Trp Gly Leu Glu Asn
       130                 135                 140
   cca acg gcc ttc gta tgc ctc gag aac acg cac aac att tgc ggt ggc      480
   Pro Thr Ala Phe Val Cys Leu Glu Asn Thr His Asn Ile Cys Gly Gly
   145                 150                 155                 160
   acc cca ctg tcg gtg gag tac acc agg gaa gcc gct gac atc gcc cga      528
   Thr Pro Leu Ser Val Glu Tyr Thr Arg Glu Ala Ala Asp Ile Ala Arg
                   165                 170                 175
   cgt gca gga gcc aaa ctc ttc atc gat ggc gca cgc atc tgg aat gct      576
   Arg Ala Gly Ala Lys Leu Phe Ile Asp Gly Ala Arg Ile Trp Asn Ala
               180                 185                 190
   gcc gtc gcg cag gac gtg acc gtg aag gat ctg tgt gaa cct gca gat      624
   Ala Val Ala Gln Asp Val Thr Val Lys Asp Leu Cys Glu Pro Ala Asp
           195                 200                 205
   gcg gtc gtg ctc tca ctc aac aag gcg ccc atc gca ccg tac ggc gct      672
   Ala Val Val Leu Ser Leu Asn Lys Ala Pro Ile Ala Pro Tyr Gly Ala
       210                 215                 220
   ctg ctc tgc ggt agc gcg gac atg atc gtg cgt gcc cgc acc gaa gcc      720
   Leu Leu Cys Gly Ser Ala Asp Met Ile Val Arg Ala Arg Thr Glu Ala
   225                 230                 235                 240
   acg cgc atc gga gcc aac cag gtt cac aaa gat ggc atc ttc gcc gct      768
   Thr Arg Ile Gly Ala Asn Gln Val His Lys Asp Gly Ile Phe Ala Ala
                   245                     250                     255
   gcc gcc atc gtc gga ttg aag gac gtc gac cag cga ctg aag gaa gac       816
   Ala Ala Ile Val Gly Leu Lys Asp Val Asp Gln Arg Leu Lys Glu Asp
               260                     265                     270
   cac cgg cgc gcg cgt cag ctg gcg gag gga ctt tca gct cac cca tcg       864
   His Arg Arg Ala Arg Gln Leu Ala Glu Gly Leu Ser Ala His Pro Ser
           275                     280                     285
   ctc gat gtc gac gct cga aac acc cga tcc aat ctc gtt cgt atc ggg       912
   Leu Asp Val Asp Ala Arg Asn Thr Arg Ser Asn Leu Val Arg Ile Gly
       290                     295                     300
   acc tca cgc act ggc gtc agc gcc ctc gag atc gcc gag gaa ctc aaa       960
   Thr Ser Arg Thr Gly Val Ser Ala Leu Glu Ile Ala Glu Glu Leu Lys
   305                     310                     315                     320
   cat caa ggc ctc ggc ttg cag gtg ctt gac cct gac acg ctc agg ctg      1008
   His Gln Gly Leu Gly Leu Gln Val Leu Asp Pro Asp Thr Leu Arg Leu
                   325                     330                     335
   gtc acc tac tgc cgc ata acc gac tgt gac atc gac gaa gct ctc ggg      1056
   Val Thr Tyr Cys Arg Ile Thr Asp Cys Asp Ile Asp Glu Ala Leu Gly
               340                     345                     350
   att ttc gaa aag gtc gtc tcc aac ctc gtg acg tcc cgc taa              1098
   Ile Phe Glu Lys Val Val Ser Asn Leu Val Thr Ser Arg
           355                     360                     365
<210> 5
   <211> 365
   <212> PRT
   <213> Rhodococcus sp.
<400> 5
   Met Pro Gly Phe Ser Gln Val Gln Trp Ser Ser Pro Pro Arg Leu Asp
   1               5                   10                  15
   Phe Arg Asn Glu Gly Arg Val Ala Pro Thr Glu Glu Met Trp Glu Ala
               20                  25                  30
   Met Arg Arg Ala Ser Glu Asn Phe Glu Met Ser Ser Phe Gly Thr Asp
           35                  40                  45
   Pro Ser Val Arg Glu Leu Glu Leu Ile Gly Ala Glu Met Thr Gly Gln
       50                  55                  60
   Glu Ala Ser Tyr Phe Leu Pro Ser Thr Thr Ala Ala Thr Leu Val Ala
   65                  70                  75                  80
   Leu Leu Ala Glu Asp Val Arg Ala Lys Gln Leu Ile Val Glu Ala Arg
                   85                  90                  95
   Ser His Leu Tyr Trp Leu Gln Arg Phe His Asp Ser Val His Ala Gly
               100                 105                 110
   Gly Arg Ala Ala Thr Ile Glu Gly Asp Lys Phe Gly Val Met Ser Leu
           115                 120                 125
   Ser Asp Ile Asp Glu Leu Ile Asn Arg Thr Val Trp Gly Leu Glu Asn
       130                 135                 140
   Pro Thr Ala Phe Val Cys Leu Glu Asn Thr His Asn Ile Cys Gly Gly
   145                 150                 155                 160
   Thr Pro Leu Ser Val Glu Tyr Thr Arg Glu Ala Ala Asp Ile Ala Arg
                   165                 170                 175
   Arg Ala Gly Ala Lys Leu Phe Ile Asp Gly Ala Arg Ile Trp Asn Ala
               180                 185                 190
   Ala Val Ala Gln Asp Val Thr Val Lys Asp Leu Cys Glu Pro Ala Asp
           195                 200                 205
   Ala Val Val Leu Ser Leu Asn Lys Ala Pro Ile Ala Pro Tyr Gly Ala
       210                 215                 220
   Leu Leu Cys Gly Ser Ala Asp Met Ile Val Arg Ala Arg Thr Glu Ala
   225                 230                 235                 240
   Thr Arg Ile Gly Ala Asn Gln Val His Lys Asp Gly Ile Phe Ala Ala
                   245                 250                 255
   Ala Ala Ile Val Gly Leu Lys Asp Val Asp Gln Arg Leu Lys Glu Asp
               260                 265                 270
   His Arg Arg Ala Arg Gln Leu Ala Glu Gly Leu Ser Ala His Pro Ser
           275                 280                 285
   Leu Asp Val Asp Ala Arg Asn Thr Arg Ser Asn Leu Val Arg Ile Gly
       290                 295                 300
   Thr Ser Arg Thr Gly Val Ser Ala Leu Glu Ile Ala Glu Glu Leu Lys
   305                 310                 315                 320
   His Gln Gly Leu Gly Leu Gln Val Leu Asp Pro Asp Thr Leu Arg Leu
                   325                 330                 335
   Val Thr Tyr Cys Arg Ile Thr Asp Cys Asp Ile Asp Glu Ala Leu Gly
               340                 345                 350
   Ile Phe Glu Lys Val Val Ser Asn Leu Val Thr Ser Arg
           355                 360                 365
<210> 6
   <211> 31
   <212> DNA
   <213> Artificial
<220>
   <223> primer (S11F)
<400> 6
   ggaagttcat atgccaggct tttcgcaggt c 31
<210> 7
   <211> 31
   <212> DNA
   <213> Artificial
<220>
   <223> primer (S12R)
<400> 7
   caggatcctt agcgggacgt cacgaggttg g 31
<210> 8
   <211> 33
   <212> DNA
   <213> Artificial
<220>
   <223> Primer (Y339SF)
<400> 8
   ctcaggctgg tcaccagctg ccgcataacc gac 33
<210> 9
   <211> 33
   <212> DNA
   <213> Artificial
<220>
   <223> Primer (Y339SR)
<400> 9
   gtcggttatg cggcagctgg tgaccagcct gag 33
<210> 10
   <211> 33
   <212> DNA
   <213> Artificial
<220>
   <223> Primer (Y339HF)
<400> 10
   ctcaggctgg tcacccattg ccgcataacc gac 33
<210> 11
   <211> 33
   <212> DNA
   <213> Artificial
<220>
   <223> Primer (Y339HR)
<400> 11
   gtcggttatg cggcaatggg tgaccagcct gag 33
<210> 12
   <211> 33
   <212> DNA
   <213> Artificial
<220>
   <223> Primer (Y339NF)
<400> 12
   ctcaggctgg tcaccaactg ccgcataacc gac 33
<210> 13
   <211> 33
   <212> DNA
   <213> Artificial
<220>
   <223> Primer (Y339NR)
<400> 13
   gtcggttatg cggcagttgg tgaccagcct gag 33
<210> 14
   <211> 33
   <212> DNA
   <213> Artificial
<220>
   <223> Primer (N19SF)
<400> 14
   cggctcgact tccggagcga ggggcgagtc gcg 33
<210> 15
   <211> 33
   <212> DNA
   <213> Artificial
<220>
   <223> Primer (N19SR)
<400> 15
   cgcgactcgc ccctcgctcc ggaagtcgag ccg 33

## Description

### TECHNICAL FIELD

The present invention relates to a method for producing a serine derivative. The present invention also relates to a protein having an enzymatic activity, which is used for the method for producing the serine derivative.

### BACKGROUND ART

Serine derivatives, for example amino acids having an optical activity at position α (β-hydroxy-α-L-amino acids, and β-hydroxy-α-D-amino acids) are substances expected to be used as intermediates of pharmaceuticals. Thus, the industrially advantageous methods for producing the serine derivative have been being developed.

As the methods for producing the serine derivative using an enzyme, for example, the method for producing α-methyl-β-hydroxyphenylalanine using a microorganism belonging genus *Xanthomonas* (Patent Document 1), and the method for producing L-serine derivatives in an optically selective manner using microorganisms belonging to genera *Ralstonia, Variovorax, Bosea* and Silicibacter or a protein derived therefrom (Patent Document 2) have been disclosed.

Patent Document 1: JP Hei-7-327688-A
Patent Document 2: International Publication WO2006/123745 Pamphlet

Schirch and Szebenyi (i.e. Current Opinion in Chemical Biology 2005, 9:482-487) concerns a review of serine hydroxymethyltransferase in particular in regard to catalytic mechanism and broad substrate specificity of this enzyme. JP20066320294 concerns method for producing beta-Hydroxyamino acid and enzyme used therefore. WO2006123745 concerns process for production of L-serine derivative and enzyme used in the process. JP3206892 concerns production of L-serine by enzymatic method.

### DISCLOSURE OF INVENTION

### PROBLEM TO BE SOLVED BY THE INVENTION

As described above, various techniques have been studied on the method for producing optically active amino acids. However, there are many kinds of the optically active amino acids and the serine derivatives. Thus, more convenient techniques or more efficient methods for generating the various serine derivatives have been required. It is an object of the present invention to provide a novel method for generating a serine derivative and optically active isomers thereof using a convenient technique, and an enzyme and the like used for the method.

### MEANS FOR SOLVING PROBLEM

The present inventors extensively studied on new methods for producing serine derivatives, and have found a new protein that catalyzes a reaction in which an α-amino acid is reacted with a certain aldehyde in a system thereof. Further it has been found that the serine derivative can be formed conveniently using this protein. The present invention is based on such findings and provides the following method for producing the serine derivative, and an enzyme and the like used for the method.

[1] A method for producing a serine derivative, comprising reacting an α-amino acid represented by formula (I): 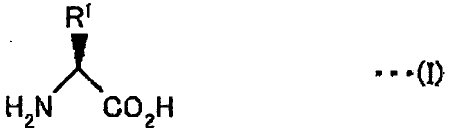 with an aldehyde represented by formula (II): 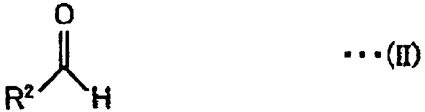 in the presence of an enzyme to form the serine derivative represented by formula (III): 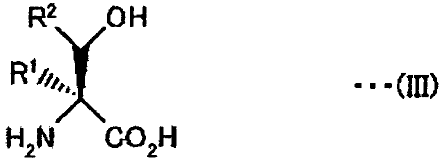 wherein R¹ of formula (I) is selected from the group consisting of an alkyl group having 1 to 7 carbon atoms, an aryl group having 6 to 14 carbon atoms, a cycloalkyl group having 3 to 10 carbon atoms, an aralkyl group having 7 to 19 carbon atoms, an alkoxyalkyl group having 2 to 11 carbon atoms, a group containing a hetero atom in the carbon skeleton thereof, and a group containing a carbon-carbon unsaturated bond in the carbon skeleton thereof, wherein these groups may be straight or branched, may have an alicyclic hydrocarbon structure and may further have a substituent,
   wherein R² of formula (II) is selected from the group consisting of hydrogen, an alkyl group having 1 to 7 carbon atoms, an aryl group having 6 to 14 carbon atoms, a cycloalkyl group having 3 to 10 carbon atoms, an aralkyl group having 7 to 19 carbon atoms, an alkoxyalkyl group having 2 to 11 carbon atoms, a group containing a hetero atom in the carbon skeleton thereof, and a group containing a carbon-carbon unsaturated bond in the carbon skeleton thereof, wherein these groups may be straight or branched, may have an alicyclic hydrocarbon structure and may further have a substituent,
   wherein R¹ of formula (III) is the same as R¹ of formula (I) and R² of formula (III) is the same as R² of formula (II);
   wherein the enzyme is one or two or more proteins selected from the group consisting of the following (A) and (B) :
   (A) a protein having the amino acid sequence of SEQ ID NO:5, and
   (B) a protein having an amino acid sequence comprising mutation of one or several amino acids which is selected from the group consisting of substitution, deletion, insertion and addition in the amino acid sequence of SEQ ID NO:5 and having an activity to catalyze the reaction to form the serine derivative represented by formula (III).
[2] The method for producing the serine derivative according to the above [1], wherein the protein (B) is a protein comprising a sequence in which tyrosine located at position 339 is substituted with any one amino acid selected from the group consisting of serine, histidine and asparagine in the amino acid sequence of SEQ ID NO:5.
[3] The method for producing the serine derivative according to the above [1] or [2], wherein the protein (B) is a protein comprising a sequence in which asparagine located at position 19 is substituted with serine in the amino acid sequence of SEQ ID NO:5.
[4] A method for producing a serine derivative, comprising reacting an α-amino acid represented by formula (I) with an aldehyde represented by formula (II) in the presence of a protein to form the serine derivative represented by formula (III),
   wherein the protein is derived from a microorganism belonging to genus *Rhodococcus* and has an activity to catalyze a reaction in which the α-amino acid represented by formula (I) is reacted with the aldehyde represented by formula (II) to form the serine derivative represented by formula (III).
[5] The method for producing the serine derivative according to any one of the above [1] to [4], wherein the microorganism producing the enzyme, the α-amino acid represented by formula (I) and the aldehyde represented by formula (II) are mixed to form the serine derivative represented by formula (III).
[6] The method for producing the serine derivative according to any one of the above [1] to [4], wherein a culture comprising the microorganism producing the enzyme, the α-amino acid represented by formula (I) and the aldehyde represented by formula (II) are mixed to form the serine derivative represented by formula (III).
[7] The method for producing the serine derivative according to any one of the above [1] to [4], wherein a treated microbial cell product of the microorganism producing the enzyme, the α-amino acid represented by formula (I) and the aldehyde represented by formula (II) are mixed to form the serine derivative represented by formula (III).
[8] The method for producing the serine derivative according to any one of the above [1] to [7], wherein the amino acid represented by formula (I) is one or two or more amino acids selected from the group consisting of phenylalanine, leucine, methionine, alanine, cysteine, tryptophan, isoleucine, cyclohexylalanine, 2-amino-n-butyric acid, 2-aminovaleric acid and 2-aminohexanoic acid.
[9] The method for producing the serine derivative according to any one of the above [1] to [7], wherein the amino acid represented by formula (I) is α-phenylalanine and the serine derivative represented by formula (III) is α-benzylserine.
[10] The method for producing the serine derivative according to any one of the above [1] to [7], wherein the amino acid represented by formula (I) is α-leucine and the serine derivative represented by formula (III) is α-isobutylserine.
[11] The method for producing the serine derivative according to any one of the above [1] to [7], wherein the amino acid represented by formula (I) is α-methionine and the serine derivative represented by formula (III) is α-methylthioethylserine.
[12] A protein which is derived from a microorganism belonging to genus *Rhodococcus* and has an activity to catalyze a reaction in which an α-amino acid represented by formula (I) is reacted with an aldehyde represented by formula (II) to form a serine derivative represented by formula (III).
[13] One or two or more proteins selected from the group consisting of the following (A) and (B):
   (A) a protein having the amino acid sequence of SEQ ID NO:5, and
   (B) a protein having an amino acid sequence comprising mutation of one or several amino acids which is selected from the group consisting of substitution, deletion, insertion and addition in the amino acid sequence of SEQ ID NO:5,
   wherein the protein has an activity to catalyze a reaction in which an α-amino acid represented by formula (I) is reacted with an aldehyde represented by formula (II) to form a serine derivative represented by formula (III).
[14] The protein according to the above [13], wherein the protein (B) comprises a sequence in which tyrosine located at position 339 is substituted with any one amino acid selected from the group consisting of serine, histidine and asparagine in the amino acid sequence of SEQ ID NO:5.
[15] The protein according to the above [14], wherein the protein (B) comprises a sequence in which asparagine located at position 19 is further substituted with serine in the amino acid sequence of SEQ ID NO:5.
[16] A polynucleotide encoding the protein according to any one of the above [12] to [15].
[17] A polynucleotide selected from the group consisting of the following (a) and (b):
   (a) a polynucleotide having the nucleotide sequence of SEQ ID NO:4, and
   (b) a polynucleotide that hybridizes with a polynucleotide having a nucleotide sequence complementary to the nucleotide sequence of SEQ ID NO:4 under a stringent condition and encodes a protein having an activity to catalyze a reaction in which an α-amino acid represented by formula (I) is reacted with an aldehyde represented by formula (II) to form a serine derivative represented by formula (III).
[18] A cell transformed with the polynucleotide according to the above [16] or [17].
[19] The cell according to the above [18], wherein the cell is a transformed *Escherichia coli* cell as a host.

### EFFECT OF THE INVENTION

According to the present invention, it is possible to produce the various serine derivatives by a convenient reaction system.

### BRIEF DESCRIPTION OF DRAWINGS

FIG. 1 is a view showing a reaction system used for the production method of the present invention; and
FIG. 2 is a view showing one embodiment of the reaction system used for the production method of the present invention.

### BEST MODES FOR CARRYING OUT THE INVENTION

Embodiments of the present invention will be described below together with best modes thereof.
Concerning various gene engineering techniques included below, various standard experiment manuals such as Molecular Cloning: A Laboratory Manual, 3rd edition. Cold Spring Harbor Press (2001) and New Gene Engineering Handbook, revised 4th edition edited by Muramatsu et al., Yodosha (2003) are available, and those skilled in the art can perform these techniques with reference to these literatures. Unless otherwise specified herein, a sequence number indicates a sequence number in Sequence Listing. An enzyme herein refers to a protein having an activity to catalyze a chemical reaction.

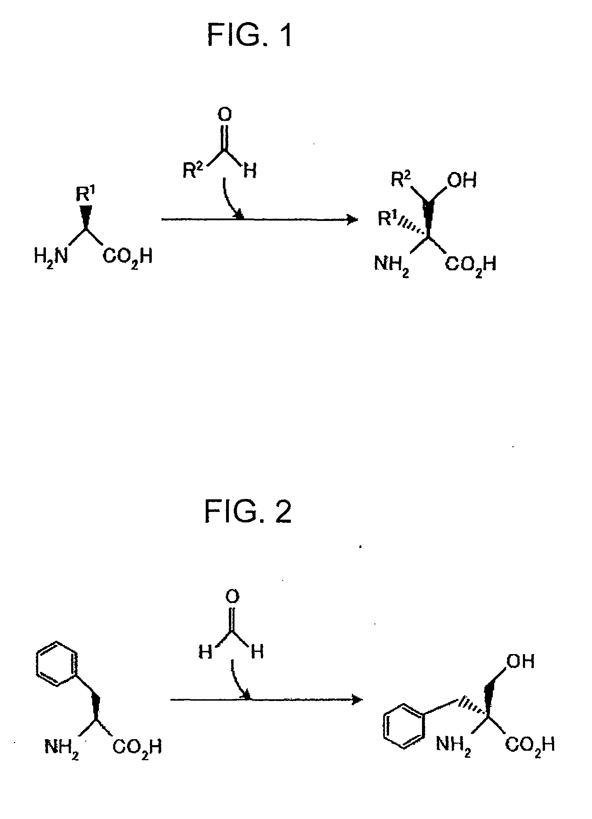

In the method for producing the serine derivative according to the present invention, the enzyme is used as a catalyst and a reaction system in which an α-amino acid of formula (I) is reacted with an aldehyde represented by formula (II) to form formula (III) is used (FIG. 1).

R¹ of formula (I) is specifically shown as follows.
When R¹ represents an alkyl group having 1 to 7 carbon atoms, methyl, ethyl, n-propyl, isopropyl, n-butyl, isobutyl, sec-butyl, tert-butyl, n-pentyl, isopentyl, neopentyl, n-hexyl and isohexyl groups are shown as specific examples thereof. Specific examples of an alkyl group having an alicyclic hydrocarbon structure in the carbon skeleton thereof may include cyclohexylmethyl.

When R¹ represents an aryl group having 6 to 14 carbon atoms, phenyl, tolyl, xylyl, biphenyl, naphthyl, anthryl and phenanthryl groups are shown as specific examples thereof.

When R¹ represents a cycloalkyl group having 3 to 10 carbon atoms, cyclopropyl, cyclobutyl, cyclopentyl, cyclohexyl, cycloheptanyl, cyclooctanyl, cyclononanyl and cyclodecanyl groups are shown as specific examples thereof.

When R¹ represents an aralkyl group having 7 to 19 carbon atoms, phenylalkyl such as benzyl, benzhydryl, phenethyl and trityl, and cinnamyl, styryl and naphthylalkyl groups are shown as specific examples thereof.

When R¹ represents an alkoxyalkyl group having 2 to 11 carbon atoms, alkyl groups having 1 to 10 carbon atoms substituted with a group selected from methoxy, ethoxy, propoxy, isopropoxy, butoxy, pentyloxy, phenoxy, heptoxy, octoxy, nonanoxy, decanoxy and undecoxy groups are shown as specific examples thereof.

R¹ may be a group comprising a hetero atom in the carbon skeleton of the above hydrocarbon. The heteroatom may include oxygen, nitrogen and sulfur.
One mode in which R¹ is the group comprising the hetero atom in the carbon skeleton thereof includes a heterocycle-containing hydrocarbon group. The heterocycle-containing hydrocarbon group is a cyclic hydrocarbon group comprising the hetero atom in a ring of a cyclic compound. The heterocycle-containing hydrocarbon group includes heteroaryl, is not limited to the presence or absence of an aromatic property and may be monocyclic or polycyclic. As the heterocycle-containing hydrocarbon group, specifically furyl, thienyl, pyridyl, piperidil, piperidino, morpholino, indolyl and imidazolyl groups, and further alkyl groups substituted with these heterocyclic groups are shown as examples.

R¹ may be a hydrocarbon group comprising a carbon-carbon unsaturated bond in the carbon skeleton thereof in the groups shown above.
Further, above R¹ may be straight or branched. Above R¹ may also have the alicyclic hydrocarbon structure in the carbon skeleton thereof. The alicyclic hydrocarbon structure may include substituent structures such as cyclopropane, cyclobutane, cyclopentane and cyclohexane, in which one or two or more hydrogen groups have been left from the carbon skeleton.
Also R¹ may be the above hydrocarbon group substituted with and/or adding one or two or more selected from halogen atoms, an alkyl group having up to 3 carbon atoms, an alkoxyl group having up to 3 carbon atoms, keto (=O), hydroxyl (-OH), thiol (-SH), amino (-NH₂), amide (-CONH₂), imino (=NH) and hydrazino (-NHNH₂) groups.

Examples of an α-amino acid shown in formula (I) may include alanine, valine, leucine, isoleucine, serine, threonine, cysteine, methionine, asparagine, glutamine, phenylalanine, tyrosine, tryptophan, aspartic acid, glutamic acid, lysine, arginine, histidine, 2-amino-n-butyric acid, cyclohexylalanine, 2-aminovaleric acid (norvaline) and 2-aminohexanoic acid (norleucine), which are all α-types, preferred are phenylalanine, leucine, methionine, alanine, cysteine, tryptophan, isoleucine, cyclohexylalanine, 2-amino-n-butyric acid, 2-aminovaleric acid and 2-aminohexanoic acid, and more preferably phenylalanine, leucine, methionine and the like.

R² of formula (II) is more specifically shown as follows.
R² may be hydrogen.
When R² represents an alkyl group having 1 to 7 carbon atoms, specific examples thereof may include methyl, ethyl, n-propyl, isopropyl, n-butyl, isobutyl, sec-butyl, tert-butyl, n-pentyl, isopentyl, neopentyl, n-hexyl and isohexyl groups. Specific examples of the alkyl group having the alicyclic hydrocarbon structure in the carbon skeleton thereof may include cyclohexylmethyl.

When R² represents an aryl group having 6 to 14 carbon atoms, specific examples thereof may include phenyl, tolyl, xylyl, biphenyl, naphthyl, anthryl and phenanthryl groups.

When R² represents a cycloalkyl group having 3 to 10 carbon atoms, specific examples thereof may include cyclopropyl, cyclobutyl, cyclopentyl, cyclohexyl, cycloheptanyl, cyclooctanyl, cyclononanyl and cyclodecanyl groups.

When R² represents an aralkyl group having 7 to 19 carbon atoms, specific examples thereof may include phenylalkyl such as benzyl, benzhydryl, phenethyl and trityl, and cinnamyl, styryl and naphthylalkyl groups.

When R² represents an alkoxyalkyl having 2 to 11 carbon atoms, specific examples thereof may include alkyl groups having 1 to 10 carbon atoms substituted with a group selected from methoxy, ethoxy, propoxy, isopropoxy, butoxy, pentyloxy, phenoxy, heptoxy, octoxy, nonanoxy, decanoxy and undecoxy groups.

R² may be the group comprising the hetero atom in the carbon skeleton thereof of the above hydrocarbon. The hetero atom may include oxygen, nitrogen and sulfur.
One mode in which R² is the group comprising the hetero atom in the carbon skeleton thereof includes the heterocycle-containing hydrocarbon group. The heterocycle-containing hydrocarbon group is the cyclic hydrocarbon group comprising the hetero atom in the ring of the cyclic compound. The heterocycle-containing hydrocarbon group includes heteroaryl, is not limited to the presence or absence of the aromatic property and may be monocyclic or polycyclic. As the heterocycle-containing hydrocarbon group, specifically furyl, thienyl, pyridyl, piperidil, piperidino, morpholino, indolyl and imidazolyl groups, and further alkyl groups substituted with these heterocyclic groups are included.

R² may be the hydrocarbon group comprising the carbon-carbon unsaturated bond in the carbon skeleton thereof in the groups shown above.
Further, above R² may be straight or branched. Above R² may also have the alicyclic hydrocarbon structure in the carbon skeleton thereof. The alicyclic hydrocarbon structure may include substituent structures such as cyclopropane, cyclobutane, cyclopentane and cyclohexane, in which one or two or more hydrogen groups have been left from the carbon skeleton.
Also R² may be the above hydrocarbon group substituted with and/or adding one or two or more selected from halogen atoms, an alkyl group having up to 3 carbon atoms, an alkoxyl group having up to 3 carbon atoms, keto (=O), hydroxyl (-OH), thiol (-SH), amino (-NH₂), amide (-CONH₂), imino (=NH) and hydrazino (-NHNH₂) groups.

As the compound represented by formula (II), preferably formaldehyde and acetaldehyde are shown as examples.

R¹ and R² of formula (III) are the same as ones of formula (I) and formula (II).

Preferable one mode in the present invention may include a system in which L-α-phenylalanine is reacted with formaldehyde to form α-benzyl-L-serine as shown in FIG. 2. As exemplified in FIG. 2, the reaction system in which without passing through 5,10-methylene tetrahydrofolate, formaldehyde is directly reacted with L-amino acid to form the L-serine derivative is used. As described above, the L-serine derivative can be obtained in the simple reaction system in the one mode of the method of the present invention. When formaldehyde is used, formaldehyde in small portions may be added sequentially. By adding formaldehyde sequentially, it is possible to inhibit the generation of byproducts and the like.

Preferable other modes in the present invention may include the system in which L-leucine is reacted with formaldehyde to form α-isobutyl-serine and the system in which L-methionine is reacted with formaldehyde to form α-methylthioethyl-serine.

A reaction temperature is preferably 10 to 60°C and more preferably 20 to 40°C. A pH value in the reaction system is preferably 4 to 10 and more preferably 6 to 9.

The serine derivative can be isolated from an enzyme reaction solution after the reaction is completed, for example, as follows.
The pH value of the enzyme reaction solution is lowered, which is sterilized by heating, as well as dissolved proteins are agglutinated. Subsequently, bacteria and the proteins are removed by means of centrifugation, filtration or UF (ultrafiltration). Inorganic salts are contained in this solution. Thus, desalting is performed in order to avoid their precipitation upon crystallization. The desalting may be performed by any of NF (nanofiltration), electric dialysis, ion exchange resin and the like.

After performing the above desalting as needed, as the reaction solution is concentrated, a crystal of the L-serine derivative is precipitated. Its feature is fine, upon separation by crystallization a high yield is not obtained due to its high solubility, and additionally its handling is often difficult due to its high viscosity. Thus, it is preferable to perform the crystallization by adding a poor solvent after preliminarily concentrating the solution as needed. The preliminary concentration may be performed until the crystal begins to precipitate. As the poor solvent to be added here, lower alcohol and acetone that are water-soluble are suitable. By combining a cooled crystallization after the crystallization with poor solvent, it is also possible to increase a crystallization rate. The crystal of the L-serine derivative can be obtained by separating this slurry and drying a wet cake.

In the present invention, the reaction of aldehyde of formula (II) and α-amino acid is performed in the presence of a certain enzyme. The enzyme that can catalyze this reaction can be obtained from, for example, a microorganism belonging to genus *Rhodococcus.* More specifically *Rhodococcus sp.* may be included, and still more specifically *Rhodococcus sp.* AJ110611 strain may be included. *Rhodococcus sp*. AJ110611 strain is a bacterial strain that has been deposited to the following Authority Depository and entrusted and managed with a given accession number of FERM BP-11042. The bacterial strain given the FERM number can be given with reference to the accession number by a certain procedure.

Name: *Rhodococcus sp.* AJ110611
Accession number: FERM BP-11042
Authority Depository: Patent Microorganism Depository, National Institute of Advanced Industrial Science and Technology
Address of Authority Depository: 1-1-1 Central No. 6 Higashi, Tsukuba-shi, Ibaraki Prefecture, Japan
Deposit Date: March 29, 2007

The accession number of FERM P-21281 had been given to the above microorganism as of the above date when it had been deposited (i.e., March 29, 2007), but the accession number of FERM BP-11042 was freshly given to it due to a transfer procedure in the authority depository, which has continuously entrusted and managed it.

*Rhodococcus sp.* AJ110611 strain had been initially deposited as *Rhodococcus percolatus* AJ110611 strain on the deposit date (i.e., March 29, 2007), but as a result of its subsequent reidentification, it was determined that this microorganism should be classified to belong to *Rhodococcus sp.* Thus, its name was changed, and this microorganism has been deposited as *Rhodococcus sp.* AJ110611 strain in the above authority depository. Only its name was changed, and this microorganism itself deposited on the above deposit date has not been changed.

More specifically, following proteins are shown as example of the enzyme used for the reaction to form the serine derivative in the present invention.
(A) a protein having the amino acid sequence of SEQ ID NO:5 (number of full length amino acid residues: 365)
(B) a protein having an amino acid sequence comprising mutation of one or several amino acids which is selected from the group consisting of substitution, deletion, insertion and addition in the amino acid sequence of SEQ ID NO:5, and having the activity to catalyze the reaction in which an α-amino acid of formula (I) is reacted with an aldehyde of formula (II) to form an serine derivative represented by formula (III).
By using the above protein, it is possible to form the serine derivative conveniently.

The protein (A) having the amino acids represented by SEQ ID NO:5 can be isolated from, for example, *Rhodococcus sp.* (AJ110611 strain). The protein (A) has a broad substrate specificity as demonstrated by following Examples.

In the present invention, a protein that is substantially the same as the protein (A) can be used. The protein shown in (B) is provided as the protein that is substantially the same as the protein (A). The term "one or several amino acids" indicates the range of the amino acid residues where the three dimensional structure and the activity of a protein are not significantly impaired although it varies depending on a position and a mutation type of the amino acid residues in a three dimensional structure of the protein. The term "one or several amino acids" indicates, for example, 1 to 100, preferably 1 to 70, more preferably 1 to 40, more preferably 1 to 20, preferably 1 to 10 and still more preferably 1 to 5 amino acids. In this regard, however, in the case of the amino acid sequence including mutation of one or several amino acids which is selected from the group consisting of one or several substitution, deletion, insertion and addition in the amino acid sequence of the protein (B), it is desirable that the enzyme having this sequence retains the enzymatic activity at about a half or more, more preferably 80% or more, still more preferably 90% or more and particularly preferably 95% or more of the protein (A) under the condition at 30°C at pH 7 to 8.

The mutation of amino acid shown in the above protein (B) is obtained by modifying a nucleotide sequence by site-directed mutagenesis such that the amino acid corresponding to a particular position in a gene encoding the present protein is substituted, deleted, inserted, added or the like. A polynucleotide having the modified nucleotide sequence as above can be obtained by conventionally known mutagenesis. Examples of the mutagenesis may include a method of treating DNA encoding the protein (A) with hydroxylamine and the like *in vitro,* and a method of treating bacteria of genus *Escherichia* carrying the DNA encoding the protein (A) with a mutagenic agent such as ultraviolet ray or N-methyl-N'-nitro-N-nitrosoguanidine (NTG) or nitrous acid which is usually used for artificial mutagenesis.

The mutation such as the substitution, the deletion, the insertion and the addition as above include differences due to species and the strain of the microorganisms and naturally occurring mutation. DNA encoding substantially the same protein as the protein (A) is obtained by expressing the DNA having the mutation as above in an appropriate cell and examining the enzymatic activity in an expressed product.

A protein having an amino acid sequence that has preferably 60% or more, more preferably 70% or more, more preferably 80% or more, more preferably 90% or more, more preferably 95% or more and still more preferably 98% or more homology to the amino acid sequence of the protein (A) is also exemplified as substantially the same protein as the protein (A). The homology of the amino acid sequences herein is calculated using a software GENETYX Ver7.0.9 (supplied from Genetyx Corporation) with setting at "unit size to compare=2" and using full length polypeptide chains encoded in ORF or calculated by a similar method to represent matching counts as a percentage.

Examples of the above protein (B) may include following mutant proteins.
(B1) a protein comprising an amino acid sequence in which tyrosine at position 339 is substituted with any one selected from the group consisting of serine, histidine and asparagine in the amino acid sequence of SEQ ID NO:5.
These mutant proteins have the higher activity to catalyze the reaction in which the amino acid of formula (I) is reacted with the aldehyde of formula (II) to form the serine derivative of formula (III) than the protein (A). Among them, the mutant protein comprising the amino acid sequence in which tyrosine at position 339 is substituted with serine has the particularly high activity. The substitution in the amino acid sequence is represented by an abbreviation of an original amino acid/a number of a mutated position/an abbreviation of a substituted amino acid in this order in some cases. For example, the substitution of the mutant protein comprising the amino acid sequence in which tyrosine at position 339 is substituted with serine can be identified by abbreviating as "Y339S" A one letter code or a three letter code are academically accepted for abbreviating the amino acid abbreviation, and the above is represented by the one letter code. The one letter code will be also used for other mutants.

Further, the other preferable mutant protein may include a following protein.
(B2) a protein comprising an amino acid sequence in which asparagine at position 19 is substituted with serine in the amino acid sequence of SEQ ID NO:5.
The mutation in the protein (B2) is abbreviated as "N19S". The mutation N19S is also involved in the activity to catalyze the reaction in which the amino acid of formula (I) is reacted with the aldehyde of formula (II) to form the serine derivative of formula (III).

More preferable mutant proteins may include modes in which both the amino acid at position 339 and the amino acid at position 19 is mutated in the amino acid sequence of SEQ ID NO:5. Namely, their combination may include following (1) to (3).
(1) "Y339S" and "N19S"
(2) "Y339H" and "N19S"
(3) "Y339N" and "N19S"
Among them, the protein comprising the amino acid sequence in which both mutations of "Y339S" and "N19S" is introduced tends to exhibit the particularly high enzymatic activity when the amino acid of formula (I) is alanine.

The present invention also provides a polynucleotide encoding the above protein (A). Multiple nucleotide sequences can be present to define one amino acid sequence due to degeneracy of codons. That is, the polynucleotide of the present invention includes a polynucleotide having a nucleotide sequence encoding the protein (A). The polynucleotide of the present invention may specifically include a polynucleotide (a) having a nucleotide sequence of SEQ ID NO:4.

The polynucleotide (a) encodes the protein (A) and can be isolated from *Rhodococcus* sp. AJ110611 strain.

Examples of other modes of the polynucleotide of the present invention may include polynucleotides encoding the above proteins (B1) and (B2) and the other mutant proteins obtained by combining the mutations. The polynucleotide encoding the mutant protein comprises a nucleotide sequence in which the sequence at position of the corresponding amino acid is substituted in accordance with a codon table in the nucleotide sequence of SEQ ID NO:4. For example, in the case of the protein having the mutation of "Y339S", the nucleotide sequence "tac" at positions 1015 to 1017 can be substituted with a serine-encoding nucleotide sequence such as "tct" in the nucleotide sequence of SEQ ID NO:4.

A method for isolation will be described. DNA having the nucleotide sequence of SEQ ID NO:4 can be obtained from chromosomal DNA or a DNA library of *Rhodococcus* sp. by PCR (polymerase chain reaction, see White, T. J. et al; Trends Genet., 5, 185 (1989)) or hybridization. Primers used for PCR can be designed based on an internal amino acid sequence determined based on the purified protein having the activity to catalyze the reaction in the method of the present invention. The primers or probes for the hybridization can also be designed based on the nucleotide sequence of SEQ ID NO:4, or the primers can also be isolated using the probes. When the primers having the sequences corresponding to a 5'-untranslated region and a 3'-untranslated region so as to sandwich a coding region are used as the primers for PCR, the full length coding region of the present protein can be amplified.

For example, the primer can be synthesized using a DNA synthesizer model 380B supplied from Applied Biosystems and using a phosphoamidite method (see Tetrahedron Letters (1981), 22, 1859) in accordance with standard methods. The PCR reaction can be performed, for example, using Gene Amp PCR System 9600 (supplied from PERKIN ELMER) or TaKaRa LA PCR in vitro Cloning Kit (supplied from Takara Bio) in accordance with the methods designated by suppliers of the manufacturers.

Substantially the same polynucleotide as the above polynucleotide (a) is also included in the polynucleotide of the present invention. Examples of substantially the same polynucleotide as the above polynucleotide (a) may include a following polynucleotide (b).

(b) a polynucleotide that hybridizes with a polynucleotide having a nucleotide sequence complementary to the nucleotide sequence of SEQ ID NO:4 under a stringent condition and encodes a protein having the activity to catalyze the reaction in which the α-amino acid of formula (I) is reacted with the aldehyde of formula (II) to form the serine derivative represented by formula (III),

For example, the probe may be used for the polynucleotide to be hybridized. In each case, the probe can be made based on the nucleotide sequence of SEQ ID NO:4 in accordance with the standard methods. Using the probe, the polynucleotide to be hybridized therewith is captured, and the objective polynucleotide can be isolated in accordance with the standard methods. For example, a DNA probe can be prepared by amplifying the nucleotide sequence cloned in a plasmid or a phage vector, cutting out the nucleotide sequence to be used as the probe using restriction enzymes and extracting it. Sites to be cut out can be controlled depending on objective DNA. Once substantially the same polynucleotide is detected, then this can be amplified by PCR in accordance with the standard methods.

The "stringent condition" refers to the condition where a so-called specific hybrid is formed whereas no nonspecific hybrid is formed. Although it is difficult to clearly quantify this condition, examples thereof may include the condition where a pair of DNA sequences with high homology, e.g., DNA sequences having the homology of 60% or more, preferably 70% or more, more preferably 80% or more, more preferably 90% or more, more preferably 95% or more and still more preferably 98% or more are hybridized whereas DNA with lower homology than that are not hybridized. The homology (%) of the nucleotide sequences is calculated using the entire ORF (including a termination codon) of each gene and using the software GENETYX Ver7.0.9 (supplied from Genetyx Corporation) with setting at "unit size to compare=6" and "pick up location=1" or calculated by the similar method to represent the matching counts as the percentage. Also another example may include the condition of hybridization at salt concentrations equivalent to 1 x SSC and 0.1% SDS at 60°C and preferably 0.1 x SSC and 0.1 SDS at 65°C, which is a washing condition of an ordinary Southern hybridization. Those in which a stop codon has occurred in an internal region of the sequence and those that have lost the activity due to the mutation of an active center are included in these genes that have hybridized under such a condition. However, these can be easily removed by ligating them to a commercially available expression vector, expressing them in an appropriate host and measuring the enzymatic activity of the expressed product by methods described later.

In the case of the above polynucleotide (b), it is desirable that the enzyme derived from this polynucleotide (b) retains the enzymatic activity at about a half or more, more preferably 80% or more and still more preferably 90% of the protein (A) having the amino acid sequence encoded by the nucleotide sequence of SEQ ID NO:4 under the condition at 30°C at pH 8.

As long as the enzyme used in the present invention exists in the reaction system in a state so that the enzyme can catalyze the reaction as above, its existence form is not particularly limited. That is, the specific existence form of the enzyme in the reaction system when the reaction is performed in the presence of the enzyme includes a culture including the microorganism producing the enzyme, a cultured microbial cell separated from the culture and a treated microbial cell product. The culture including the microorganism indicates things obtained by culturing the microorganism, and more specifically indicates the cultured microbial cell, a medium used for culturing the microorganism, a substance produced by the cultured microorganism, and a mixture thereof. The cultured microbial cell may be washed to use as a washed microbial cell. The treated microbial cell product includes products obtained by disrupting, lysing and lyophilizing the microbial cell, a cell-free extract and a crude protein collected by further treating the microbial cell, and a purified protein obtained by further purifying this. A partially purified protein obtained by various purification methods and an immobilized protein obtained by immobilizing this by a covalent bond method, an adsorption method or an inclusive method may be used as the purified protein. Depending on the microorganism to be used, some of it is lysed in a cultivation in some cases. Thus, a culture supernatant can also be utilized as an enzyme-containing matter in this case.

Subsequently, the method for producing the protein of the present invention, and the method for making a recombinant body and a transformant used for it will be described by taking the above protein (A) as an example. The same methods are also feasible for the other mutant proteins.

The transformant that expresses the above protein (A) can be made by making a recombinant polynucleotide in which the polynucleotide having any of the above nucleotide sequences is incorporated and using this. For example, the transformant that expresses the protein (A) can be obtained by making recombinant DNA in which DNA having the nucleotide sequence shown in SEQ ID NO:4 is incorporated and introducing it into an appropriate host. As the host for expressing the particular protein by DNA having the nucleotide sequence of SEQ ID NO:4, various prokaryotic cells including bacteria belonging to genus *Escherichia* such as *Escherichia coli* and genus *Corynebacterium, Bacillus subtilis,* and various eukaryotic cells including *Saccharomyces cerevisiae*, *Pichia stipitis* and *Aspergillus oryzae* can be used. The serine derivative can be produced inexpensively and conveniently on a large scale by the use of the host, handling such as cultivation of which is convenient and which can be cultured without requiring expensive components.

The recombinant DNA used for introducing the DNA having the nucleotide sequence of SEQ ID NO:4 into the host can be prepared by inserting the DNA having that sequence into a vector that meets a type of the host to express the protein in a form so that the protein encoded by the DNA can be expressed. As a promoter to express the protein, when the promoter inherent to the gene encoding the above enzyme derived from *Rhodococcus sp* works in the host, that promoter can be used. If necessary, the other promoter that works in the host may be ligated to the DNA such as SEQ ID NO:4 to express the protein under the control of that promoter.

Transformation methods for introducing the recombinant DNA into the host cell may include D. M. Morrison's method (Methods in Enzymology 68, 326 (1979)) or a method of increasing a permeability of DNA by treating a recipient bacterium with calcium chloride (Mandel, M. and Higa, A., J. Mol. Biol., 53, 159 (1970)).

In the case of producing the objective protein on a large scale using the recombinant DNA technology, a preferable mode thereof may include formation of an inclusion body of the protein, in which the protein is aggregated in the transformant producing the protein, as one preferable embodiment. The advantages of this expression production method may include the protection of the objective protein from digestion by proteases present in the microbial cells, and convenient purification of the objective protein that may be performed by disruption of the microbial cells and the following centrifugation. To obtain the active protein from the protein inclusion body, a series of the manipulations such as solubilization and activity regeneration is required, and thus the manipulations is more complicate than those in the case of directly producing the active protein. However, when a protein which affects microbial cell growth is produced on a large scale in the microbial cells, effects thereof can be inhibited by accumulating the protein as the inactive inclusion body in the microbial cells.

As the methods for producing the objective protein on a large scale as the inclusion body, methods of expressing the protein alone under control of a strong promoter, as well as methods of expressing the objective protein as a fusion protein with a protein known to be expressed in a large amount are available.

A strain generally used for the expression of a xenogenic gene can be used for the host to be transformed, and can be selected from *Escherichia coli* K12 strain subspecies, JM109 strain, DH5α strain, HB101 strain and BL21 (DE3) strain. Methods of performing the transformation and methods of selecting the transformant are also described in Molecular Cloning: A Laboratory Manual, 3rd edition, Cold Spring Harbor press (2001/01/15). Hereinafter, methods of making transformed *Escherichia coli* and producing a certain enzyme using this will be more specifically described as one example.

The promoter typically used in the production of a xenogenic protein in *Escherichia coli* can be used as the promoter to express the DNA encoding the protein having the catalytic activity used in the present invention. Examples thereof may include strong promoters such as T7 promoter, lac promoter, trp promoter, trc promoter, tac promoter, PR and PL promoters of lambda phage, and T5 promoter. As the vector, for example, pUC19, pUC18, pBR322, pHSG299, pHSG298, pHSG399, pHSG398, RSF1010, pACYC177, pACYC184, pMW119, pMW118, pMW219, pMW218, pQE30 and derivatives thereof may be used. As the other vector, the vector of phage DNA may be utilized. An expression vector that contains the promoter and can express an inserted DNA sequence may be used.

In order to produce the protein used in the present invention as a fusion protein inclusion body, a gene encoding another protein, preferably a hydrophilic peptide is ligated to an upstream or downstream of the protein, whereby a fusion protein gene is produced. Such a gene encoding the other protein may be the one which increases an accumulation amount of the fusion protein and enhances solubility of the fusion protein after steps of modification and regeneration, and example thereof may include T7 gene 10, β-galactosidase gene, dehydrofolate reductase gene, interferon y gene, interleukin-2 gene, prochymosin gene and the like as candidates.

These genes are ligated to the gene encoding the protein so that reading frames of codons are matched. Such a ligation may be performed by ligation at an appropriate restriction enzyme site, or by utilization of synthetic DNA with an appropriate sequence.

In order to augment a production amount, it is sometimes preferable to ligate a transcription termination sequence i.e., a terminator to the downstream of the fusion protein gene. This terminator may be include T7 terminator, fd phage terminator, T4 terminator, a terminator of a tetracycline resistant gene, a terminator of an Escherichia coli trpA gene, or the like.

As the vector to introduce the gene encoding the protein having the catalytic activity or the fusion protein into *Escherichia coli,* so-called multiple copying types are preferable. Plasmids having a replication origin derived from ColE1, such as pUC type plasmids, pBR322 type plasmids or derivatives thereof may be included. As used herein, the "derivatives," mean those in which modification is given to the plasmids by the substitution, the deletion, the insertion, the addition and/or an inversion of nucleotides. The modification as referred to herein may also include modification by mutagenic treatments by mutagenic agents and UV irradiation or natural mutations.

It is preferred that the vector has a marker such as an ampicillin resistant gene for selecting the transformant. As such a plasmid, the expression vectors carrying strong promoters are commercially available (for example, pUC types (supplied from Takara Bio Co., Ltd.), pPROK types (supplied from Clontech), pKK233-2 (supplied from Clontech) and the like).

A DNA fragment obtained by ligating the promoter, the gene encoding the objective protein having a certain activity or the fusion protein of the objective protein and the other protein, and the terminator in some cases, in this order is ligated to the vector DNA to obtain recombinant DNA.

Using the resulting recombinant DNA, *Escherichia coli* is transformed, and Cultivation of this *Escherichia coli* results in expression and production of a certain protein or the fusion protein thereof.

In the case of expressing as the fusion protein, the fusion protein may be composed so as to be able to cleave the objective protein therefrom using a restriction protease which recognizes a sequence of blood coagulation factor Xa, kallikrein or the like which is not present in the objective protein.

The production media to be used may include the media usually used for culturing *Escherichia coli,* such as M9-casamino acid medium and LB medium. Culture conditions and production induction conditions may be appropriately selected depending on types of the vector marker, the promoter, the host bacterium and the like.

The objective protein or the fusion protein including this may be recovered by the following method. When the objective protein or the fusion protein thereof is solubilized in the microbial cells, the microbial cells can be collected and then disrupted or lysed, to obtain a crude enzyme solution. If necessary, the objective protein or the fusion protein thereof can further be purified in accordance with ordinary techniques such as precipitation, filtration and column chromatography. The purification can be utilized in accordance with methods utilizing an antibody against the objective protein or the fusion protein. When the protein inclusion body is formed, this is solubilized with a denaturing agent, and the objective protein can be obtained by removing the denaturing agent by dialysis and the like.

### EXAMPLES

The present invention will be illustrated in more detail with reference to the following Examples, but the invention is not limited thereto. *Rhodococcus sp.* AJ110611 strain used in the following Examples had been designated as *Rhodococcus percolatus* AJ110611 strain as its name on the deposit date (i.e., March 29, 2007) in the authority depository, but as a result of the subsequent reidentification, this microorganism was determined to be classified to belong to *Rhodococcus sp.*

### <Example 1> Detection of 2-benzylserine

### hydroxymethyltransferase activity (identification of activity in wild strain)

*Rhodococcus sp.* (AJ110611 strain) was cultured in an agar medium prepared with Nutrient Broth (Difco) at 30°C for 24 hours. A liquid medium (pH 7.0) containing 3 mL of 0.2% α-benzyl-DL-serine and 0.17% Yeast Nitrogen Base w/o amino acid and ammonium sulfate (Difco) was inoculated with one loopful of microbial cells of the cultured microorganism, and then cultured with shaking at 30°C at 120 reciprocations/min for 24 hours. After the cultivation, the microbial cells were centrifuged, and washed twice with 50 mM potassium phosphate buffer (pH 7.4) containing 1 mL of 0.1 mM pyridoxal phosphate. A microbial cell suspension in a total amount of 0.3 ml was prepared using the washed microbial cells and 50 mM potassium phosphate buffer (pH 7.4) containing 0.1 mM pyridoxal phosphate. The resulting microbial cell suspension was sonicated and disrupted at 4°C. A supernatant obtained by centrifuging the microbial cell suspension after being sonicated was dialyzed against 50 mM potassium phosphate buffer (pH 7.4) containing 0.1 mM pyridoxal phosphate to make a cell-free extract solution.

A reaction mixture in a total amount of 0.1 mL obtained by adding 0.03 mL of the cell-free extract solution to a reaction solution A having a composition of 50 mM potassium phosphate buffer (pH 7.4), 10 mM α-benzyl-DL-serine and 0.1 mM pyridoxal phosphate was reacted at 30°C for 10 minutes. After 10 minutes, the reaction was stopped by mixing 0.1 mL of an alkali reagent (5 N potassium hydroxide) attached in a formaldehyde kit, Test Wako (Wako Pure Chemicals Industries Ltd.).

Subsequently, a detection reaction of formaldehyde was performed and an absorbance at 550 nm was measured (E11) in accordance with a manual attached to the kit. As a control, the absorbance of a reaction solution obtained by the reaction using water instead of α-benzyl-DL-serine in the above reaction solution A was likewise measured (E10). An absorbance change (EΔ1=E11-E10) specific for α-benzyl-DL-serine was calculated from the measured values E11 and E10, and a value of 1.25 was obtained. From the above result, the 2-benzylserine hydroxymethyltransferase activity was confirmed in the cell-free extract solution prepared from *Rhodococcus* sp. (AJ110611 strain).

### <Example 2> Purification of 2-benzylserine hydroxymethyltransferase derived from Rhodococcus sp. (AJ110611 strain)

### (1) Preparation of cell-free extract solution

*Rhodococcus sp.* (AJ110611 strain) was cultured in the agar medium prepared with Nutrient Broth (Difco) at 30°C for 24 hours. 50 mL of Nutrient Broth liquid medium in a 500 mL Sakaguchi flask was inoculated with microbial cells of the cultured microorganism, and then cultured with shaking at 25°C at 120 reciprocations/min for 19 hours. Twenty Sakaguchi flasks in which 50 mL of the liquid medium (pH 7.0) containing 0.2% α-benzyl-DL-serine and 0.17% Yeast Nitrogen Base w/o amino acid and ammonium sulfate (Difco) had been added were prepared, and the liquid medium in the Sakaguchi flask was inoculated with 0.25 mL of the resulting culture medium. After the inoculation, the liquid medium was cultured with shaking at 25°C at 120 reciprocations/min for 24 hours. The resulting microbial cells were collected by centrifugation (8,000×g, 10 minutes), and washed twice with 10 mL of 50 mM potassium phosphate buffer A (pH 7.4) containing 0.1 mM pyridoxal phosphate. The microbial cells were further washed twice with 25 mM potassium phosphate buffer B (pH 7.4) containing 0.02 mM pyridoxal phosphate and 1 mM EDTA to prepare 40 mL of a microbial cell suspension. The microbial cells were disrupted by sonication. A supernatant obtained by centrifugation (8,000xg, 20 minutes) was further subjected to ultracentrifugation (200,000xg, 30 minutes). The resulting supernatant was dialyzed against the potassium phosphate buffer B to obtain a cell-free extract solution.

### (2) Anion-exchange chromatography

The cell-free extract solution obtained in above (1) was applied to Q-Sepharose-HP 16/10 column (supplied from Amersham Bioscience) previously equilibrated with the 25 mM potassium phosphate buffer B (pH 7.4) containing 0.02 mM pyridoxal phosphate and 1 mM EDTA, and the enzyme was eluted with a linear concentration gradient of 0 to 1 M sodium chloride. The enzymatic activity was measured using an obtained fraction as an enzyme source in accordance with the method in Example 1, and the activity of 2-benzylserine hydroxymethyltransferase was detected in the fractions corresponding to 0.3 to 0.4 M of sodium chloride.

### (3) Hydrophobic interaction chromatography

The fraction having the enzymatic activity obtained in above (2) was dialyzed against the 25 mM potassium phosphate buffer B (pH 7.4) containing 0.02 mM pyridoxal phosphate and 1 mM EDTA, and mixed with the buffer B containing 2 M ammonium sulfate in an equal amount. The resulting mixture was applied to Phenyl-Sepharose-HP 16/10 column (supplied from Amersham Bioscience) previously equilibrated with the buffer B (pH 7.4) containing 1 M ammonium sulfate, and the enzyme was eluted with the linear concentration gradient of 1 to 0 M ammonium sulfate. The enzymatic activity was measured using the obtained fraction as the enzyme source in accordance with the method in Example 1, and the activity of 2-benzylserine hydroxymethyltransferase was detected in the fractions corresponding to 0.7 to 0.6 M ammonium sulfate.

### (4) Gel filtration chromatography

The fraction having the enzymatic activity obtained in above (3) was concentrated, applied to Superdex-200 16/60 column (supplied from Amersham Bioscience) previously equilibrated with the 25 mM potassium phosphate buffer (pH 7.4) containing 0.02 mM pyridoxal phosphate and 1 mM EDTA, and the enzyme was eluted. The enzymatic activity was measured using the obtained fractions as the enzyme source in accordance with the method in Example 1, and the activity of 2-benzylserine hydroxymethyltransferase was detected in the fractions corresponding to 64 to 68 ml of eluted volumes.

### (5) Anion-exchange chromatography

The fraction having the enzymatic activity obtained in above (4) was applied to MonoQ HR5/5 column (supplied from Amersham Bioscience) previously equilibrated with the 25 mM potassium phosphate buffer (pH 7.4) containing 0.02 mM pyridoxal phosphate and 1 mM EDTA, and the enzyme was eluted with the linear concentration gradient of 0 to 0.7 M sodium chloride. The enzymatic activity was measured using the obtained fractions as the enzyme source in accordance with the method in Example 1, and the activity of 2-benzylserine hydroxymethyltransferase was detected in the fractions corresponding to 0.45 to 0.55 M of sodium chloride.

A specific activity in the active fraction of the enzyme obtained by passing through the above steps (1) to (5) was 1.82 U/mg. The resulting purified enzyme was subjected to SDS polyacrylamide electrophoresis and the gel was stained with SimplyBlue SafeStain (Invitrogen), and a uniform band was detected at a position corresponding to a molecular weight of about 40 kDa.

### <Example 3> Sequencing and gene cloning of 2-benzylserine hydroxymethyltransferase derived from Rhodococcus sp. (AJ110611 strain)

The purified enzyme corresponding to an amount of 100 pmol prepared in Example 2 was run on the SDS-polyacrylamide electrophoresis, and then transferred onto a PVDF membrane. The resulting sample was subjected to a protein sequencer PPSQ-21A supplied from Shimadzu Corporation, and 23 amino acid residues in an N-terminal amino acid sequence were determined (SEQ ID NO:1).

Then, 5 µg of genomic DNA from *Rhodococcus sp.* (AJ110611 strain) was cleaved with PstI (75 U), and ligated to a PstI cassette in accordance with the method described in the manual of TaKaRa LA PCR in vitro Cloning Kit (supplied from TaKaRa). PCR was performed using the ligated mix as a template and a combination of a cassette primer C1 and a primer SEQA (SEQ ID NO:2) (94°C: 30 seconds, 50°C: 30 seconds, 72°C: 4 minutes; 30 cycles). Then, the second PCR was performed using this PCR solution as the template and using a cassette primer C2 and a primer SEQB (SEQ ID NO:3) (94°C: 30 seconds, 50°C: 30 seconds, 72°C: 4 minutes; 30 cycles). A fragment having a length about 0.6 kb, amplification of which had been confirmed was ligated to pT7Blue Teasy (Novagen). *Escherichia coli* JM109 was transformed with the plasmid in which the amplified fragment had been inserted.

A nucleotide sequence of the about 0.6 kb fragment was determined, and the nucleotide sequence encoding the N-terminal amino acid sequence of the objective protein was found. Chromosomal DNA treated with various restriction enzymes were analyzed by Southern blotting using this about 0.6 kb gene fragment as a probe, and an about 5 kb fragment treated with SacI was confirmed to have a positive signal. Then, the chromosomal DNA treated with SacI was run on an agarose gel electrophoresis to purify the about 5 kb fragment, which was then ligated to a SacI site of pUC18. *Escherichia coli* JM109 was transformed with this reaction solution to make a library. Colony hybridization was performed using the above probe, a positive colony was obtained, and a plasmid was extracted. The resulting plasmid was designated as pUCRHMT5K. The nucleotide sequence inserted in pUCRHMT5K was sequenced, and an ORF encoding 365 amino acid residues was present (SEQ ID NO:4).

### <Example 4> Expression of 2-benzylserine hydroxymethyltransferase gene derived from Rhodococcus sp. (AJ110611 strain) in Escherichia coli (preparation of the expressing bacterium JM109/pTrp4)

1.1 kb of the ORF region of the 2-benzylserine hydroxymethyltransferase gene was amplified by PCR with pUCRHMT5K as the template using the primer S11F (SEQ ID NO:6) and the primer S12R (SEQ ID NO:7). The amplified fragment treated with NdeI/BamHI was ligated to pTrp4 vector (Journal of Molecular Catalysis B: Enzymatic, 2005, 32, 205-211) previously treated with NdeI/BamHI. *Escherichia coli* JM109 was transformed with the resulting vector to obtain a transformant having a plasmid (pTrp4RHMT) containing an objective gene fragment. This transformant was designated as JM109/pTrp4RHMT.

3 ml of an LB medium containing 100 mg/l ampicillin was inoculated with one loopful of JM109/pTrp4RHMT cultured overnight in an LB agar medium containing 100 mg/l ampicillin, and then was cultured at 37°C for 16 hours. The resulting microbial cells were collected by centrifugation, and washed with 50 mM potassium phosphate buffer (pH 7.4) containing 0.1 mM pyridoxal phosphate. A microbial cell suspension was prepared using 0.3 mL of the same buffer. The microbial cell suspension was sonicated to disrupt the microbial cells. A supernatant obtained by centrifugation (18,000×g, 10 minutes, 4°C) was used as a cell-free extract solution. The 2-benzylserine hydroxymethyltransferase activity in the resulting cell-free extract solution was measured, and was 0.017 U/mg. The activity in the cell-free extract solution obtained in the same manner as in the above method except using JM/pTrp4, the transformant obtained by transforming JM109 with pTrp4 in which no PCR product had been inserted was detection limit or less.

### <Example 5> Expression of 2-benzylserine hydroxymethyltransferase gene derived from Rhodococcus sp. (AJ110611 strain) in Escherichia coli (preparation of the expressing bacterium JM109/pSFNRHMT)

The plasmid pTrp4RHMT made in Example 4 was treated with BamHI, subsequently blunted in accordance with the method described in the manual of TaKaRa DNA Blunting Kit (TaKaRa), and then treated with NdeI to prepare the 2-benzylserine hydroxymethyltransferase gene. Subsequently, pSFN vector (International Publication WO2006/075486 Pamphlet) was treated with PstI, then blunted in accordance with the method described in the manual of TaKaRa DNA Blunting Kit (TaKaRa), and treated with NdeI. This vector was ligated to the gene previously prepared. *Escherichia coli* JM109 was transformed with this ligation product to obtain a transformant having a plasmid containing the objective gene fragment (pSFNRHMT). This transformant was designated as JM109/pSFNRHMT.

3 ml of the LB medium containing 100 mg/l ampicillin was inoculated with one loopful of JM109/pSFNRHMT cultured overnight in the LB agar medium containing 100 mg/l ampicillin, and then was cultured at 37°C for 20 hours. The resulting microbial cells were collected by centrifugation, and washed with 50 mM potassium phosphate buffer (pH 7.4) containing 0.1 mM pyridoxal phosphate. A microbial cell suspension was prepared using 0.3 mL of the same buffer. The microbial cell suspension was sonicated to disrupt the microbial cells. A supernatant obtained by centrifugation (18,000xg, 10 minutes, 4°C) was used as a cell-free extract solution. The 2-benzylserine hydroxymethyltransferase activity in the resulting cell-free extract solution was measured, and was 0.014 U/mg.

### <Example 6> Reaction of generating α-benzyl-L-serine by 2-benzylserine hydroxymethyltransferase derived from Rhodococcus sp. (AJ110611 strain)-BnS synthesis reaction by wild strain

50 µl of the purified enzyme solution prepared in Example 2 was added to a solution composed of 10 mM formaldehyde, 30 mM L-phenylalanine, 0.1 mM pyridoxal phosphate and 50 mM potassium phosphate buffer (pH 7.4), and the mixture was reacted at 30°C for 30 minutes. A special grade formaldehyde solution [code number: 16223-55] supplied from Nacalai Tesque Inc. was used as formaldehyde. After completion of the reaction, 100 µl of 4 mM copper sulfate was added to 100 µl of the reaction mixture, which was then analyzed by HPLC using SumichiralOA-5000 (Sumika Chemical Analysis Service Ltd.) (Mobile phase: aqueous solution of 2 mM copper sulfate (10% isopropyl alcohol), column temperature: 30°C, flow rate: 1 ml/minute, detection: UV 230 nm). As a result, it was confirmed that 8.9 mM α-benzyl-L-serine had been formed.

### <Example 7> Stereological determination of benzylserine formed by enzymatic reaction

In order to synthesize α-benzyl-L-serine, in accordance with a literature (Angew. Chem. Int. Ed. 2004, 43, 2382-2385), 1.41 ml of triethylamine was added to a methylene chloride solution in which 939.2 mg of ethyl benzimidate hydrochloride and 1 g of L-serine-tert-butyl ester hydrochloride had been dissolved. The mixture was heated and refluxed for 2.5 hours, and stirred at room temperature for 19 hours. 50 ml of methylene chloride was added to the reaction mixture, which was then washed twice with 20 mL of water, and dried on magnesium sulfate. Magnesium sulfate was removed, and then the solvent was evaporated. A residue was purified by silica gel chromatography (hexane/ethyl acetate, 5/1 to 4/1). After evaporating the solvent, 759 mg of 2-phenyl-2-oxazoline-4-carboxylate tert-butyl ester was yielded. 0.12 ml of benzyl bromide was added to a toluene solution in which 52.4 mg of 2-phenyl-2-oxazoline-4-carboxylate tert-butyl ester, 56.1 mg of potassium hydroxide and 9.55 mg of (S,S)-3,4,5-trifluorophenyl-NAS bromide (Wako Pure Chemical Industries Ltd., code number: 201-16401) had been suspended. The resulting mixture was stirred at 0°C for 8 hours. 20 ml of ethyl acetate was added to the reaction mixture, which was then washed twice with 5 mL of water, and dried on magnesium sulfate. Magnesium sulfate was removed, and then the solvent was evaporated. A residue was purified by silica gel chromatography (hexane/ethyl acetate, 8/1 to 5/1). After evaporating the solvent, 51 mg of 4-benzyl 2-phenyl-2-oxazoline-4-carboxylate tert-butyl ester was yielded. This was analyzed by HPLC using CHIRALPAK OD-H (4.6×250mm) (Mobile phase: hexane/isopropyl alcohol=99/1, column temperature: room temperature, flow rate: 1 ml/minute, detection: UV 210 nm), and its optical purity was 98.4%e.e. 1 ml of ethanol and 0.5 ml of 6 N sodium hydroxide solution were added to 42 mg of 4-benzyl 2-phenyl-2-oxazoline-4-carboxylic acid tert-butyl ester, which was then heated and refluxed for 1 hour. Then 1 ml of 6 N HCl was added, and the reaction mixture was heated and refluxed for 2 hours. The reaction mixture was neutralized and impurities were removed to obtain a solution containing α-benzyl-L-serine.

The resulting solution containing α-benzyl-L-serine was analyzed by HPLC using SumichiralOA-5000 (Sumika Chemical Analysis Service Ltd.) (Mobile phase: aqueous solution of 2 mM copper sulfate (10% isopropyl alcohol), column temperature: 30°C, flow rate: 1 ml/minute, detection: UV 230 nm). As a result, it was found that this has the same retention time as α-benzylserine obtained from the enzymatic reaction. From the above results, it was found that as α-benzylserine obtained from the enzymatic reaction was α-benzyl-L-serine.

### <Example 8> Generation of α-benzyl-L-serine by Escherichia coli that expresses 2-benzylserine hydroxymethyltransferase gene derived from Rhodococcus sp. (AJ110611 strain) (BnS synthesis by the expressing bacterium JM109/pTrp4RHMT: CFE)

3 ml of the LB medium containing 100 mg/l ampicillin was inoculated with one loopful of JM109/pTrp4RHMT cultured overnight in the LB agar medium containing 100 mg/l ampicillin, and then was cultured at 37°C for 16 hours. The resulting microbial cells were collected by centrifugation, and washed with 50 mM potassium phosphate buffer (pH 7.4) containing 0.1 mM pyridoxal phosphate. A microbial cell suspension was prepared using 0.3 ml of the same buffer. The microbial cell suspension was sonicated to disrupt the microbial cells. A supernatant obtained by centrifugation (18,000xg, 10 minutes, 4°C) was used as a cell-free extract solution. 30 µl of the resulting cell-free extract solution was added to a solution composed of 10 mM formaldehyde, 30 mM L-phenylalanine, 0.1 mM pyridoxal phosphate and 50 mM potassium phosphate buffer (pH 7.4), and the mixture was reacted at 30°C for 30 minutes. The special grade formaldehyde solution [code number: 16223-55] supplied from Nacalai Tesque Inc. was used as formaldehyde. After the completion of the reaction, 100 µl of 4 mM copper sulfate was added to 100 µl of the reaction mixture.

Then, 100 µl of water was added to 100 µl of the resulting solution, which was then analyzed by HPLC using SumichiralOA-5000 (Sumika Chemical Analysis Service Ltd.) (Mobile phase: aqueous solution of 2 mM copper sulfate (10% isopropyl alcohol), column temperature: 30°C, flow rate: 1 ml/minute, detection: UV 230 nm). As a result, it was confirmed that 5.2 mM α-benzyl-L-serine had been formed. The activity in the cell-free extract solution obtained in the same manner as in the above method except using JM109/pTrp4, the transformant obtained by transforming JM109 with pTrp4 in which no PCR product had been inserted was detection limit or less.

### <Example 9> Generation of α-benzyl-L-serine by Escherichia coli that expresses 2-benzylserine hydroxymethyltransferase gene derived from Rhodococcus sp. (AJ110611 strain) (BnS synthesis by the expressing bacterium JM109/pSFNRHMT: CFE)

3 ml of the LB medium containing 100 mg/l ampicillin was inoculated with one loopful of JM109/pSFNRHMT cultured overnight in the LB agar medium containing 100 mg/l ampicillin, and then was cultured at 37°C for 17 hours. The resulting microbial cells were collected by centrifugation, and washed with 50 mM potassium phosphate buffer (pH 7.4) containing 0.1 mM pyridoxal phosphate. A microbial cell suspension was prepared using 0.3 ml of the same buffer. The microbial cell suspension was sonicated to disrupt the microbial cells. A supernatant obtained by centrifugation (18,000×g, 10 minutes, 4°C) was used as a cell-free extract solution. 30 µl of the resulting cell-free extract solution was added to the solution composed of 10 mM formaldehyde, 30 mM L-phenylalanine, 0.1 mM pyridoxal phosphate and 50 mM potassium phosphate buffer (pH 7.4), and the mixture was reacted at 30°C for one hour. The special grade formaldehyde solution [code number: 16223-55] supplied from Nacalai Tesque Inc. was used as formaldehyde. After the completion of the reaction, 100 µl of 4 mM copper sulfate was added to 100 µl of the reaction mixture, which was then analyzed by HPLC using SumichiralOA-5000 (Sumika Chemical Analysis Service Ltd.) (Mobile phase: aqueous solution of 2 mM copper sulfate (10% isopropyl alcohol), column temperature: 30°C, flow rate: 1 ml/minute, detection: UV 230 nm). As a result, it was confirmed that 7.8 mM α-benzyl-L-serine had been formed.

### <Example 10> Generation of α-benzyl-L-serine by Escherichia coli that expresses 2-benzylserine hydroxymethyltransferase gene derived from Rhodococcus sp. (AJ110611 strain) (BnS synthesis by the expressing bacterium pSFNRHMT: cell)

3 ml of the LB medium containing 100 mg/l ampicillin was inoculated with one loopful of JM109/pSFNRHMT cultured overnight in the LB agar medium containing 100 mg/l ampicillin, and then was cultured at 37°C for 16.5 hours. 50 ml of the LB medium containing 100 mg/l ampicillin in a 500 ml Sakaguchi flask was inoculated with 2.5 ml of the resulting microbial cell medium, and then cultured at 37°C at 120 reciprocations/min for 17 hours. Microbial cells were collected from 10 ml of the resulting cultured medium by centrifugation (8,000xg, 10 minutes), and washed with 10 ml of 50 mM potassium phosphate buffer (pH 7.4) containing 0.1 mM pyridoxal phosphate. A microbial cell suspension was prepared using 10 mL of the same buffer. Then 100 µL of the solution composed of 10 mM formaldehyde, 30 mM L-phenylalanine, 0.1 mM pyridoxal phosphate and 50 mM potassium phosphate buffer (pH 7.4) was added to the microbial cells collected from 1 mL of the microbial cell suspension by centrifugation (18,000×g, 10 minutes). The mixture was reacted at 30°C for 10 minutes. The special grade formaldehyde solution [code number: 16223-55] supplied from Nacalai Tesque Inc. was used as formaldehyde. After the completion of the reaction, 100 µl of 4 mM copper sulfate was added to the reaction mixture, which was then centrifuged (18,000×g, 5 minutes) to separate the microbial cells. Then, the supernatant was analyzed by HPLC using SumichiralOA-5000 (Sumika Chemical Analysis Service Ltd.) (Mobile phase: aqueous solution of 2 mM copper sulfate (10% isopropyl alcohol), column temperature: 30°C, flow rate: 1 ml/minute, detection: UV 230 nm). As a result, it was confirmed that 6.2 mM α-benzyl-L-serine had been formed.

### <Example 11> Generation of α-isobutyl-serine by Escherichia coli that expresses 2-benzylserine hydroxymethyltransferase gene derived from Rhodococcus sp. (AJ110611 strain) (iBuS synthesis by the expressing bacterium pSFNRHMT: cell)

50 ml of the LB medium containing 100 mg/l ampicillin in a 500 mL Sakaguchi flask was inoculated with one loopful of JM109/pSFNRHMT cultured overnight in the LB agar medium containing 100 mg/l ampicillin, and was cultured with shaking at 37°C at 120 reciprocations/min overnight. Microbial cells were collected from 10 mL of the resulting cultured medium by centrifugation (8,000xg, 10 minutes), and washed with 10 mL of 50 mM potassium phosphate buffer (pH 7.4) containing 0.1 mM pyridoxal phosphate. A microbial cell suspension was prepared using 10 ml of the same buffer. Then 100 µl of a solution composed of 10 mM formaldehyde, 30 mM L-leucine, 0.1 mM pyridoxal phosphate and 50 mM potassium phosphate buffer (pH 7.4) was added to the microbial cells collected from 1 ml of the microbial cell suspension by centrifugation (18,000×g, 10 minutes). The mixture was reacted at 30°C for 30 minutes. The special grade formaldehyde solution [code number: 16223-55] supplied from Nacalai Tesque Inc. was used as formaldehyde. After the completion of the reaction, 100 µl of 4 mM copper sulfate was added to the reaction mixture, which was then centrifuged (18,000×g, 5 minutes) to separate the microbial cells. Then, the supernatant was analyzed by HPLC using SumichiralOA-5000 (Sumika Chemical Analysis Service Ltd.) (Mobile phase: aqueous solution of 2 mM copper sulfate (5% isopropyl alcohol), column temperature: 30°C, flow rate: 1 ml/minute, detection: UV 230 nm). As a result, it was confirmed that 0.6 mM α-isobutyl-serine had been formed.

### <Reference Example> Synthesis of α-methylthioethyl-serine

In accordance with the literature (J. Peptide Sci., 2001, 7, 619-625), 10 g of DL-methionine was added to 20 ml of water and 11.5 ml of 6 N NaOH, and dissolved therein. With keeping pH at 10.5 to 11.5, 8.6 ml of benzoyl chloride was added thereto, and the mixture was stirred at room temperature for two hours. The resulting reaction mixture was filtrated and then washed with diethyl ether. Concentrated hydrochloric acid was gradually added to adjust pH to 1 to precipitate a crystal. The crystal was filtrated, washed with water, and dried under reduced pressure to obtain 16.2 g of N-benzoylmethionine. 24 ml of acetic anhydride was added to 4.9 g of obtained N-benzoylmethionine, the solution was heated at 100°C for 4 hours, then cooled to room temperature, and the solvent was evaporated. Adding 24 ml of toluene to a residue and evaporating the solvent were repeated twice. A residue was dissolved in 2.5 ml of pyridine, 10 ml of 35% formaldehyde solution was added, and stirred at room temperature for 17 hours. 40 ml of water was added to the resulting reaction solution, which was then extracted with 40 ml of ethyl acetate. The extract was washed with water and saturated brine, and dried on magnesium sulfate. Magnesium sulfate was removed and the solvent was evaporated. A residue was purified by silica gel chromatography (hexane/ethyl acetate 4/1 to 1/1). After evaporating the solvent, 2.56 g of 5-benzoylamino-5-methylthioethyl-4-oxo-1,3-dioxane was obtained. 7.5 ml of 6 N hydrochloric acid was added to 1.06 g of the obtained 5-benzoylamino-5-methylthioethyl-4-oxo-1,3-dioxane, which was then heated and refluxed for 7 hours. The reaction solution was filtrated, and then the solvent was evaporated. 5 ml of water was added to a residue, which was then purified using an ion exchange resin (Amberlite 120). After evaporating the solvent, the resulting crystal was recrystallized from water/ethanol, and dried under reduced pressure to obtain 129 mg of α-methylthioethyl-serine. NMR of this compound was measured, and almost agreed with values described in the literature.

### <Example 12> Synthesis of α-methylthioethyl-serine by Escherichia coli that expresses 2-benzylserine hydroxymethyltransferase gene derived from Rhodococcus sp. (AJ110611 strain) (HmMet synthesis by the expressing bacterium pSFNRHMT: cell)

50 ml of the LB medium containing 100 mg/l ampicillin in a 500 ml Sakaguchi flask was inoculated with one loopful of JM109/pSFNRHMT cultured overnight in the LB agar medium containing 100 mg/l ampicillin, and then was cultured with shaking at 37°C at 120 reciprocations/min overnight. Microbial cells were collected from 10 ml of the resulting cultured medium by centrifugation (8,000×g, 10 minutes), and washed with 10 ml of 50 mM potassium phosphate buffer (pH 7.4) containing 0.1 mM pyridoxal phosphate. A microbial cell suspension was prepared using 10 ml of the same buffer. Then 100 µl of a solution composed of 10 mM formaldehyde, 30 mM L-methionine, 0.1 mM pyridoxal phosphate and 50 mM potassium phosphate buffer (pH 7.4) was added to the microbial cells collected from 1 ml of the microbial cell suspension by centrifugation (18,000xg, 10 minutes). The mixture was reacted at 30°C for 30 minutes. The special grade formaldehyde solution [code number: 16223-55] supplied from Nacalai Tesque Inc. was used as formaldehyde. After the completion of the reaction, 100 µl of 4 mM copper sulfate was added to the reaction mixture, which was then centrifuged (18,000×g, 5 minutes) to separate the microbial cells. Then, the supernatant was analyzed by HPLC using SumichiralOA-5000 (Sumika Chemical Analysis Service Ltd.) (Mobile phase: aqueous solution of 2 mM copper sulfate (2% isopropyl alcohol), column temperature: 30°C, flow rate: 1 ml/minute, detection: UV 230 nm). As a result, it was confirmed that 2.8 mM α-methylthioethyl-serine had been formed.

### <Example 13> Production of the mutant strains Y339S, Y339H and Y339N

pSFNRHMT made in Example 5 was used as the template in site-directed mutagenesis using PCR in order to construct mutant 2-benzylserine hydroxymethyltransferase. The mutation of Y339S was introduced using Quick change Site-Directed Mutagenesis Kit supplied from Stratagene (USA) in accordance with a protocol of its manufacturer and using the primer Y339SF (SEQ ID NO:8) and the primer Y339SR (SEQ ID NO:9), which both corresponded to the Y339S mutant enzyme. Likewise, the mutation of Y339H was introduced using the primer Y339HF (SEQ ID NO:10) and the primer Y339HR (SEQ ID NO:11), and the mutation of Y339N was introduced using the primer Y339NF (SEQ ID NO:12) and the primer Y339NR (SEQ ID NO:13). All of these primers corresponded to the mutant enzymes, respectively. *Escherichia coli* JM109 was transformed with a PCR product, and transformants having a plasmid containing an objective gene fragment were obtained. These transformants were designated as JM109/pSFNRHMT-Y339S, JM109/pSFNRHMT-Y339H and JM109/pSFNRHMT-Y339N, respectively.

### <Example 14> Reaction of the mutant strains Y339S, Y339H and Y339N

3 ml of the LB medium containing 100 mg/l ampicillin was inoculated with JM109/pSFNRHMT-Y339S, JM109/pSFNRHMT-Y339H and JM109/pSFNRHMT-Y339N, respectively. The inoculated media were cultured at 37°C overnight. The resulting microbial cells were collected by centrifugation (18,000×g, 10 minutes, 4°C), and washed with 50 mM potassium phosphate buffer (pH 7.4) containing 0.1 mM pyridoxal phosphate. Microbial cell suspensions were prepared using 0.3 ml of the same buffer. The microbial cell suspensions were sonicated to disrupt the microbial cells. Supernatants obtained by centrifugation (18,000×g, 10 minutes, 4°C) were used as cell-free extract solutions. 0.02 ml of the cell-free extract solution was added to the solution composed of 50 mM potassium phosphate buffer (pH 7.4), 10 mM α-benzyl-DL-serine and 0.1 mM pyridoxal phosphate. The reaction mixture in a total amount of 0.06 ml was reacted at 30°C for 10 minutes. After 10 minutes, the reaction was stopped by mixing 0.06 ml of the alkali reagent (5 N potassium hydroxide) attached in the formaldehyde kit, Test Wako (Wako Pure Chemicals Industries Ltd.). Subsequently, the detection reaction of formaldehyde was performed and the absorbance at 550 nm was measured in accordance with the manual attached to the kit. The amount of formed formaldehyde was obtained and the activity was calculated. Results are shown in Table 1.

**(Table 1)**

| Strain | U/mg |
|---|---|
| JM109/pSFNRHMT | 0.013 |
| JM109/pSFNRHMT-Y339S | 0.043 |
| JM109/pSFNRHMT-Y339H | 0.033 |
| JM109/pSFNRHMT-Y339N | 0.036 |

### <Example 15> Production of the mutant strain Y339S-N19S

pSFNRHMT-Y339S made in Example 13 was used as the template in the site-directed mutagenesis using PCR in order to construct mutant 2-benzylserine hydroxymethyltransferase. The mutation was introduced using Quick change Site-Directed Mutagenesis Kit supplied from Stratagene (USA) in accordance with the protocol of its manufacturer and using the primer N19SF (SEQ ID NO:14) and the primer N19SR (SEQ ID NO:15), which both corresponded to the mutant enzyme. *Escherichia coli* JM109 was transformed with a PCR product, and a transformant having a plasmid containing an objective gene fragment was obtained. This transformant was designated as JM109/pSFNRHMT-Y339S-N19S. 3ml of the LB medium containing 100 mg/l ampicillin was inoculated with the prepared JM109/pSFNRHMT-Y339S-N19S. The inoculated medium was cultured at 37°C overnight. The resulting microbial cells were collected by centrifugation (18,000×g, 10 minutes, 4°C), and washed with 50 mM potassium phosphate buffer (pH 7.4) containing 0.1 mM pyridoxal phosphate. A microbial cell suspension was prepared using 0.3 ml of the same buffer. The microbial cell suspension was sonicated to disrupt the microbial cells. A supernatant obtained by centrifugation (18,000xg, 10 minutes, 4°C) was used as a cell-free extract solution. 0.005 ml of the cell-free extract solution was added to the solution composed of 50 mM potassium phosphate buffer (pH 7.4), 10 mM α-benzyl-DL-serine and 0.1 mM pyridoxal phosphate. The reaction mixture in a total amount of 0.06 ml was reacted at 30°C for 5 minutes. After 5 minutes, the reaction was stopped by mixing 0.06 ml of the alkali reagent (5 N potassium hydroxide) attached in the formaldehyde kit, Test Wako (Wako Pure Chemicals Industries Ltd.). Subsequently, the detection reaction of formaldehyde was performed and the absorbance at 550 nm was measured in accordance with the manual attached to the kit. The amount of the formed formaldehyde was obtained and the activity was calculated. The results are shown in Table 2.

**(Table 2)**

| Strain | U/mg |
|---|---|
| JM109/pSFNRHMT | 0.03 |
| JM109/pSFNRHMT-Y339S | 0.34 |
| JM109/pSFNRHMT-Y339S-N19S | 0.17 |

### <Example 16> Synthesis of BnS using JM109/pSFNRHMT-Y339S

3 ml of the LB medium containing 100 mg/l ampicillin was inoculated with one loopful of JM109/pSFNRHMT-Y339S cultured overnight in the LB agar medium containing 100 mg/l ampicillin, and cultured at 37°C for 16.5 hours. 50 ml of the LB medium containing 100 mg/l ampicillin in a 500 ml Sakaguchi flask was inoculated with 2.5 ml of the resulting microbial cell medium, and cultured at 37°C at 120 reciprocations/min for 17 hours. The microbial cells were collected from 10 ml of the resulting cultured medium by centrifugation (8,000xg, 10 minutes), and washed with 10 ml of 50 mM potassium phosphate buffer (pH 7.4) containing 0.1 mM pyridoxal phosphate. A microbial cell suspension was prepared using 10 ml of the same buffer. Then 100 µl of the solution composed of 10 mM formaldehyde, 30 mM L-phenylalanine, 0.1 mM pyridoxal phosphate and 50 mM potassium phosphate buffer (pH 7.4) was added to the microbial cells collected from 0.2 ml of the microbial cell suspension by centrifugation (18,000xg, 10 minutes). The mixture was reacted at 30°C for 10 minutes. The special grade formaldehyde solution [code number: 16223-55] supplied from Nacalai Tesque Inc. was used as formaldehyde. After the completion of the reaction, 100 µl of 4 mM copper sulfate was added to the reaction mixture, which was then centrifuged (18,000xg, 5 minutes) to separate the microbial cells. Then, the supernatant was analyzed by HPLC using SumichiralOA-5000 (Sumika Chemical Analysis Service Ltd.) (Mobile phase: aqueous solution of 2 mM copper sulfate (10% isopropyl alcohol), column temperature: 30°C, flow rate: 1 ml/minute, detection: UV 230 nm). As a result, it was confirmed that α-benzyl-L-serine had been quantitatively formed.

### <Example 17> Synthesis of iBus using JM109/pSFNRHMT-Y339S

50 ml of the LB medium containing 100 mg/l ampicillin in a 500 ml Sakaguchi flask was inoculated with one loopful of JM109/pSFNRHMT-Y339S cultured overnight in the LB agar medium containing 100 mg/l ampicillin, and cultured with shaking at 37°C at 120 reciprocations/min overnight. The microbial cells were collected from 10 ml of the resulting cultured medium by centrifugation (8,000xg, 10 minutes), and washed with 10 ml of 50 mM potassium phosphate buffer (pH 7.4) containing 0.1 mM pyridoxal phosphate. A microbial cell suspension was prepared using 10 ml of the same buffer. Then 100 µl of the solution composed of 10 mM formaldehyde, 30 mM L-leucine, 0.1 mM pyridoxal phosphate and 50 mM potassium phosphate buffer (pH 7.4) was added to the microbial cells collected from 1 ml of the microbial cell suspension by centrifugation (18,000×g, 10 minutes). The mixture was reacted at 30°C for 30 minutes. The special grade formaldehyde solution [code number: 16223-55] supplied from Nacalai Tesque Inc. was used as formaldehyde. After the completion of the reaction, 100 µl of 4 mM copper sulfate was added to the reaction mixture, which was then centrifuged (18,000xg, 5 minutes) to separate the microbial cells. Then, the supernatant was analyzed by HPLC using SumichiralOA-5000 (Sumika Chemical Analysis Service Ltd.) (Mobile phase: aqueous solution of 2 mM copper sulfate (5% isopropyl alcohol), column temperature: 30°C, flow rate: 1 ml/minute, detection: UV 230 nm). As a result, it was confirmed that 4.6 mM α-isobutyl-serine had been formed.

### <Example 18> Synthesis of BnS using JM109/pSFNRHMT-Y339-N19S

350 ml of TB medium (12 g/l of trypton, 24 g/l of yeast extract, 2.3 g/l of potassium dihydrogen phosphate, 12.5 g/l of dipotassium hydrogen phosphate, 4 g/l of glycerol) containing 100 mg/l of ampicillin was inoculated with JM109/pSFNRHMT-Y339S-N19S cultured overnight in the LB agar medium containing 100 mg/l ampicillin, and cultured at 37°C for 17 hours. The microbial cells were collected by centrifugation (8,000xg, 10 minutes), washed with 50 ml of 50 mM potassium phosphate buffer (pH 7.4) containing 0.1 mM pyridoxal phosphate, and added to the reaction solution (30 mM L-phenylalanine, 45 mM formaldehyde, 0.1 mM pyridoxal phosphate and 50 mM potassium phosphate buffer (pH 7.4), 200 ml). The reaction mixture was stirred at 30°C for 5 hours, and then, 100 µl of 4 mM copper sulfate and 80 µl of water were added to 20 µl of the reaction mixture, which was then centrifuged (18,000×g, 5 minutes) to separate the microbial cells. Then, the supernatant was analyzed by HPLC using SumichiralOA-5000 (Sumika Chemical Analysis Service Ltd.) (Mobile phase: aqueous solution of 2 mM copper sulfate (10% isopropyl alcohol), column temperature: 30°C, flow rate: 1 ml/minute, detection: UV 230 nm). As a result, it was confirmed that 28.2 mM α-benzyl-L-serine had been formed.

### <Example 19> Synthesis of BnS using JM109/pSFNRHMT-Y339-N19S

700 ml of TB medium (12 g/l of trypton, 24 g/l of yeast extract, 2.3 g/l of potassium dihydrogen phosphate, 12.5 g/l of dipotassium hydrogen phosphate, 4 g/l of glycerol) was inoculated with JM109/pSFNRHMT-Y339S-N19S cultured overnight in the LB agar medium containing 100 mg/l ampicillin, and cultured at 37°C for 17 hours. The cultured medium was divided into two (350 ml). The microbial cells were collected from each of them by centrifugation (8,000×g, 10 minutes), and washed with 50 ml of 50 mM potassium phosphate buffer (pH 7.4) containing 0.1 mM pyridoxal phosphate. The microbial cells corresponding to 350 ml of the culture medium were added to the reaction solution (60 mM L-phenylalanine, 90 mM formaldehyde, 0.1 mM pyridoxal phosphate and 50 mM potassium phosphate buffer (pH 7.4), 200 ml), which was then stirred at 30°C for 6.5 hours. Then, the microbial cells corresponding to another 350 ml of the culture medium were added thereto, which was further stirred for 15 hours. After the completion of the reaction, 100 µl of 4 mM copper sulfate and 80 µl of water were added to 20 µl of the reaction mixture, which was then centrifuged (18,000xg, 5 minutes) to separate the microbial cells. Then, the supernatant was analyzed by HPLC using SumichiralOA-5000 (Sumika Chemical Analysis Service Ltd.) (Mobile phase: aqueous solution of 2 mM copper sulfate (10% isopropyl alcohol), column temperature: 30°C, flow rate: 1 ml/minute, detection: UV 230 nm). As a result, it was confirmed that 57.6 mM α-benzyl-L-serine had been formed.

### <Example 20> Purification of α-benzyl-L-serine

The microbial cells were simply removed from the reaction mixture obtained in Example 18 by centrifugation (8,000×g, 10 minutes). Sulfuric acid was added to the resulting solution to adjust pH to 3, and dissolved proteins were heated and agglutinated at 50°C for 2 hours. This was filtrated with 0.2 µm MF. This solution was fed to a column filled with 40 ml of a strongly acidic cation exchange resin (Amberlite IR120 supplied from Aldrich) to adsorb α-benzyl-L-serine. Water in an amount of 2.5 times the amount of resin was run through the column to wash the resin, then the peptide was eluted using 1 M ammonia water and an eluant was collected every 10 ml. The obtained second to tenth fractions were concentrated under reduced pressure to almost remove ammonia. The obtained crystal was dried at 50°C under reduced pressure overnight to yield 1.57 g (93wt%) of α-benzyl-L-serine as the crystal.

### <Example 21> Purification of α-benzyl-L-serine

841 mg (22wt%) of a crude crystal containing 184 mg of α-benzyl-L-serine, which had been obtained by the purification using the strongly acidic cation exchange resin (Amberlite IR120 supplied from Aldrich) like Example 20 was dissolved in about 5 ml of water. This solution was fed to the column filled with 17 ml of a synthetic adsorbent (SP207 supplied from Mitsubishi Chemical Corporation), and eluted with the water. Fractions containing α-benzyl-L-serine were collected, concentrated under reduced pressure, and dried at 50°C under reduced pressure overnight to yield 174 mg (97wt%) of α-benzyl-L-serine as the crystal. An optical rotation of this crystal was measured, and was [α]²⁰_{D} = +16.8 (C 0.79, H₂O) that agreed with the value described in the literature. Value in the literature [α]²⁰_{D} = +16.4 (C 0.81, H₂O) (Synlett 1997, 253.).

### <Example 22> Synthesis of α-methylthioethyl-L-serine

750 ml of the TB medium (12 g/l of trypton, 24 g/l of yeast extract, 2.3 g/l of potassium dihydrogen phosphate, 12.5 g/l of dipotassium hydrogen phosphate, and 4 g/l of glycerol) containing 100 mg/l of ampicillin was inoculated with JM109/pSFNRHMT-Y339S-N19S cultured overnight in the LB agar medium containing 100 mg/l of ampicillin, and cultured at 37°C for 17 hours. The microbial cells were collected by centrifugation (8,000×g, 10 minutes), washed with 50 ml of 50 mM potassium phosphate buffer (pH 7.4) containing 0.1 mM pyridoxal phosphate, and added to a reaction solution (60 mM L-methionine, 90 mM formaldehyde, 0.1 mM pyridoxal phosphate and 50 mM potassium phosphate buffer (pH 7.4), 200 ml). The reaction mixture was stirred at 30°C for 23 hours, and then 100 µl of 4 mM copper sulfate and 80 µl of water were added to 20 µl of the reaction mixture, which was then centrifuged (18,000xg, 5 minutes) to separate the microbial cells. Then, the supernatant was analyzed by HPLC using SumichiralOA-5000 (Sumika Chemical Analysis Service Ltd.) (Mobile phase: aqueous solution of 2 mM copper sulfate (2% isopropyl alcohol), column temperature: 30°C, flow rate: 1 ml/minute, detection: UV 230 nm). As a result, it was confirmed that 46 mM α-methylthioethyl-L-serine had been formed.

### <Example 23> Purification of α-methylthioethyl-L-serine

750 ml of the TB medium (12 g/l of trypton, 24 g/l of yeast extract, 2.3 g/l of potassium dihydrogen phosphate, 12.5 g/l of dipotassium hydrogen phosphate, and 4 g/l of glycerol) containing 100 mg/l of ampicillin was inoculated with JM109/pSFNRHMT-Y339S-N19S cultured overnight in the LB agar medium containing 100 mg/l ampicillin, and cultured at 37°C for 17 hours. The cultured medium was divided into two (350 ml + 400 ml). The microbial cells were collected from each of them by centrifugation (8,000×g, 10 minutes), and washed with 50 ml of 50 mM potassium phosphate buffer (pH 7.4) containing 0.1 mM pyridoxal phosphate. The microbial cells corresponding to 350 ml of the culture medium were added to the reaction solution (60 mM L-methionine, 90 mM formaldehyde, 0.1 mM pyridoxal phosphate and 50 mM potassium phosphate buffer (pH 7.4), 200 ml), which was then stirred at 30°C for 4 hours. The microbial cells corresponding to 400 ml of the cultured medium were added and the mixture was stirred for additional 18 hours. The microbial cells and formaldehyde were added depending on residual amounts. The microbial cells were simply removed from the reaction mixture by centrifugation (8,000×g, 10 minutes). Sulfuric acid was added to the resulting solution to adjust pH to 2.5, and dissolved proteins were heated and agglutinated at 50°C for one hour. Insoluble matters were removed by centrifugation (8,000xg, 10 minutes). The solution was filtrated with 0.45 µm MF. This solution was fed to the column filled with 40 ml of the strongly acidic cation exchange resin (Amberlite IR120 supplied from Aldrich) to adsorb α-methylthioethyl-L-serine. Water in an amount of 2.5 times the amount of resin was run through the column to wash the resin, then the peptide was eluted using 1 M ammonia water and an eluant was collected every 10 ml. The obtained second to tenth fractions were concentrated under reduced pressure to almost remove ammonia. The obtained crystal was dried at 50°C under reduced pressure overnight to yield 1.40 g (98wt%) of α-methylthioethyl-L-serine as the crystal. The optical rotation of this crystal was measured, and was [α]²⁰_{D} = -21.7 (C 1, 5N HCl) that agreed with the value described in the literature. Value in the literature [α]²⁰_{D} = -10.5 (C 1, 5N HCl) (J. Peptide Sci., 2001, 7, 619.)

### <Example 24> Synthesis of α-methylserine

50 ml of the LB medium containing 100 mg/l ampicillin in a 500 ml Sakaguchi flask was inoculated with JM109/pSFNRHMT-Y339S cultured overnight in the LB agar medium containing 100 mg/l ampicillin, and cultured at 37°C at 120 reciprocations/min for 17 hours. Microbial cells were collected by centrifugation (8,000xg, 10 minutes), and washed with 30 ml of 50 mM potassium phosphate buffer (pH 7.4) containing 0.1 mM pyridoxal phosphate. A microbial cell suspension was prepared using 50 ml of the same buffer. Then 100 µl of a solution composed of 10 mM formaldehyde, 30 mM L-alanine, 0.1 mM pyridoxal phosphate and 50 mM potassium phosphate buffer (pH 7.4) was added to the microbial cells collected from 2 ml of the microbial cell suspension by centrifugation (18,000xg, 10 minutes). The mixture was reacted at 30°C for one hour. After the completion of the reaction, an ESI-MS analysis was performed using a supernatant obtained by centrifugation (18,000×g, 5 minutes, 4°C), and a molecular ion peak of α-methylserine was observed.

### <Example 25> Synthesis of α-carbamoylmethylserine

50 ml of the LB medium containing 100 mg/l ampicillin in a 500 ml Sakaguchi flask was inoculated with JM109/pSFNRHMT-Y339S cultured overnight in the LB agar medium containing 100 mg/l ampicillin, and cultured at 37°C at 120 reciprocations/min for 17 hours. Microbial cells were collected by centrifugation (8,000×g, 10 minutes), and washed with 30 ml of 50 mM potassium phosphate buffer (pH 7.4) containing 0.1 mM pyridoxal phosphate. A microbial cell suspension was prepared using 50 ml of the same buffer. Then 100 µl of a solution composed of 10 mM formaldehyde, 30 mM L-asparagine, 0.1 mM pyridoxal phosphate and 50 mM potassium phosphate buffer (pH 7.4) was added to the microbial cells collected from 2 ml of the microbial cell suspension by centrifugation (18,000×g, 10 minutes). The mixture was reacted at 30°C for one hour. After the completion of the reaction, the ESI-MS analysis was performed using a supernatant obtained by centrifugation (18,000×g, 5 minutes, 4°C), and a molecular ion peak of α-carbamoylmethylserine was observed.

### <Example 26> Synthesis of α-thiomethylserine

50 ml of the LB medium containing 100 mg/l ampicillin in a 500 ml Sakaguchi flask was inoculated with JM109/pSFNRHMT-Y3395 cultured overnight in the LB agar medium containing 100 mg/l ampicillin, and cultured at 37°C at 120 reciprocations/min for 17 hours. Microbial cells were collected by centrifugation (8,000xg, 10 minutes), and washed with 30 ml of 50 mM potassium phosphate buffer (pH 7.4) containing 0.1 mM pyridoxal phosphate. A microbial cell suspension was prepared using 50 ml of the same buffer. Then 100 µl of a solution composed of 10 mM formaldehyde, 30 mM L-cysteine, 0.1 mM pyridoxal phosphate and 50 mM potassium phosphate buffer (pH 7.4) was added to the microbial cells collected from 2 ml of the microbial cell suspension by centrifugation (18,000xg, 10 minutes). The mixture was reacted at 30°C for one hour. After the completion of the reaction, the ESI-MS analysis was performed using a supernatant obtained by centrifugation (18,000xg, 5 minutes, 4°C), and a molecular ion peak of α-thiomethylserine was observed.

### <Example 27> Synthesis of α-indolemethylserine

50 ml of the LB medium containing 100 mg/l ampicillin in a 500 ml Sakaguchi flask was inoculated with JM109/pSFNRHMT-Y339S cultured overnight in the LB agar medium containing 100 mg/l ampicillin, and cultured at 37°C at 120 reciprocations/min for 17 hours. Microbial cells were collected by centrifugation (8,000xg, 10 minutes), and washed with 30 ml of 50 mM potassium phosphate buffer (pH 7.4) containing 0.1 mM pyridoxal phosphate. A microbial cell suspension was prepared using 50 ml of the same buffer. Then 100 µl of a solution composed of 10 mM formaldehyde, 30 mM L-tryptophan, 0.1 mM pyridoxal phosphate and 50 mM potassium phosphate buffer (pH 7.4) was added to the microbial cells collected from 2 ml of the microbial cell suspension by centrifugation (18,000xg, 10 minutes). The mixture was reacted at 30°C for one hour. After the completion of the reaction, the ESI-MS analysis was performed using a supernatant obtained by centrifugation (18,000xg, 5 minutes, 4°C), and a molecular ion peak of α-indolemethylserine was observed.

### <Example 28> Synthesis of α-sec-butylserine

50 ml of the LB medium containing 100 mg/l ampicillin in a 500 ml Sakaguchi flask was inoculated with JM109/pSFNRHMT-Y339S cultured overnight in the LB agar medium containing 100 mg/l ampicillin, and cultured at 37°C at 120 reciprocations/min for 17 hours. Microbial cells were collected by centrifugation (8,000xg, 10 minutes), and washed with 30 ml of 50 mM potassium phosphate buffer (pH 7.4) containing 0.1 mM pyridoxal phosphate. A microbial cell suspension was prepared using 50 ml of the same buffer. Then 100 µl of a solution composed of 10 mM formaldehyde, 30 mM L-isoleucine, 0.1 mM pyridoxal phosphate and 50 mM potassium phosphate buffer (pH 7.4) was added to the microbial cells collected from 2 ml of the microbial cell suspension by centrifugation (18,000xg, 10 minutes). The mixture was reacted at 30°C for one hour. After the completion of the reaction, the ESI-MS analysis was performed using a supernatant obtained by centrifugation (18,000xg, 5 minutes, 4°C), and a molecular ion peak of α-sec-butylserine was observed.

### <Example 29> Synthesis of α-p-hydroxy-benzylserine

50 ml of the TB medium (12 g/l of trypton, 24 g/l of yeast extract, 2.3 g/l of potassium dihydrogen phosphate, 12.5 g/l of dipotassium hydrogen phosphate, and 4 g/l of glycerol) containing 100 mg/l of ampicillin was inoculated with JM109/pSFNRHMT-Y339S-N19S cultured overnight in the LB agar medium containing 100 mg/l ampicillin, and cultured at 37°C for 18 hours. The microbial cells were collected by centrifugation, washed with 50 ml of 50 mM potassium phosphate buffer (pH 7.4) containing 0.1 mM pyridoxal phosphate. A microbial cell suspension was prepared using 5 ml of the same buffer. The microbial cell suspension was sonicated to disrupt the microbial cells. A supernatant obtained by centrifugation (18,000xg, 10 minutes, 4°C) was used as a cell-free extract solution. 53.3 µl of the obtained cell-free extract solution was added to a solution composed of 1 mM L-tyrosine, 3 mM formaldehyde, 0.1 mM pyridoxal phosphate and 50 mM potassium phosphate buffer (pH 7.4). The mixture was reacted at 30°C for one hour. After the completion of the reaction, the ESI-MS analysis was performed using a supernatant obtained by centrifugation (18,000xg, 5 minutes, 4°C), and a molecular ion peak of α-p-hydroxy-benzylserine was observed.

### <Example 30> Synthesis of α-cyclohexylmethylserine

50 ml of the LB medium containing 100 mg/l ampicillin was inoculated with JM109/pSFNRHMT-Y339S-N19S cultured overnight in the LB agar medium containing 100 mg/l ampicillin, and cultured at 37°C for 17 hours. The microbial cells were collected by centrifugation, and washed with 50 ml of 50 mM potassium phosphate buffer (pH 7.4) containing 0.1 mM pyridoxal phosphate. A microbial cell suspension was prepared using 5 ml of the same buffer. The microbial cell suspension was sonicated to disrupt the microbial cells. A supernatant obtained by centrifugation (18,000xg, 10 minutes, 4°C) was used as a cell-free extract solution. 59 µl of the obtained cell-free extract solution was added to a solution composed of 12.5 mM cyclohexylalanine, 3 mM formaldehyde, 0.1 mM pyridoxal phosphate and 50 mM potassium phosphate buffer (pH 7.4). The mixture was reacted at 30°C for 30 minutes. After the completion of the reaction, the ESI-MS analysis was performed using a supernatant obtained by centrifugation (18,000xg, 5 minutes, 4°C), and a molecular ion peak of α-cyclohexylmethylserine was observed.

### <Example 31> Synthesis of methylserine using JM109/pSFNRHMT-Y339S-N19S

50 ml of the TB medium (12 g/l of trypton, 24 g/l of yeast extract, 2.3 g/l of potassium dihydrogen phosphate, 12.5 g/l of dipotassium hydrogen phosphate, and 4 g/l of glycerol) containing 100 mg/l of ampicillin in a 500 ml Sakaguchi flask was inoculated with JM109/pSFNRHMT-Y339S-N19S cultured overnight in the LB agar medium containing 100 mg/l of ampicillin, and cultured at 37°C at 120 reciprocations/min for 16 hours. The microbial cells were collected from 10 ml of the cultured medium by centrifugation (8,000×g, 10 minutes), and then washed with 10 ml of 50 mM potassium phosphate buffer (pH 7.4) containing 0.1 mM pyridoxal phosphate. A microbial cell suspension was prepared using 10 ml of the same buffer. Then 100 µl of the solution composed of 30 mM formaldehyde, 30 mM L-alanine, 0.1 mM pyridoxal phosphate and 50 mM potassium phosphate buffer (pH 7.4) was added to the microbial cells collected from 1 ml of the microbial cell suspension by centrifugation (18,000xg, 10 minutes). The mixture was reacted at 30°C for one hour. The special grade formaldehyde solution [code number: 16223-55] supplied from Nacalai Tesque Inc. was used as formaldehyde. After the completion of the reaction, 100 µl of 4 mM copper sulfate was added to the reaction mixture, which was then centrifuged (18,000xg, 5 minutes) to separate the microbial cells. 100 µl of water was added to 100 µl of the resulting solution, which was then analyzed by HPLC using SumichiralOA-5000 (Sumika Chemical Analysis Service Ltd.) (Mobile phase: aqueous solution of 0.5 mM copper sulfate, column temperature: 30°C, flow rate: 1 ml/minute, detection: UV 230 nm). As a result, it was confirmed that 3.4 mM α-methyl-L-serine had been formed.

### <Example 32>. Synthesis of α-ethylserine

50 ml of the LB medium containing 100 mg/l of ampicillin in a 500 ml Sakaguchi flask was inoculated with JM109/pSFNRHMT-Y339S cultured overnight in the LB agar medium containing 100 mg/l ampicillin, and cultured at 37°C at 120 reciprocations/min for 17 hours. The microbial cells were collected from 10 ml of the cultured medium by centrifugation (8,000×g, 10 minutes), and then washed with 10 ml of 50 mM potassium phosphate buffer (pH 7.4) containing 0.1 mM pyridoxal phosphate. A microbial cell suspension was prepared using 10 ml of the same buffer. Then 100 µl of a solution composed of 10 mM formaldehyde, 30 mM S-2-amino-n-butyric acid, 0.1 mM pyridoxal phosphate and 50 mM potassium phosphate buffer (pH 7.4) was added to the microbial cells collected from 2 ml of the microbial cell suspension by centrifugation (18,000×g, 5 minutes, 4°C). The mixture was reacted at 30°C for one hour. After the completion of the reaction, the ESI-MS analysis was performed using a supernatant obtained by centrifugation (18,000×g, 5 minutes, 4°C), and a molecular ion peak of α-ethylserine was observed.

### <Example 33> Synthesis of α-propylserine

50 ml of the LB medium containing 100 mg/l of ampicillin in a 500 ml Sakaguchi flask was inoculated with JM109/pSFNRHMT-Y339S cultured overnight in the LB agar medium containing 100 mg/l ampicillin, and cultured at 37°C at 120 reciprocations/min for 17 hours. The microbial cells were collected from 10 ml of the cultured medium by centrifugation (8,000×g, 10 minutes), and then washed with 10 ml of 50 mM potassium phosphate buffer (pH 7.4) containing 0.1 mM pyridoxal phosphate. A microbial cell suspension was prepared using 10 ml of the same buffer. Then 100 µl of a solution composed of 10 mM formaldehyde, 30 mM L-norvaline, 0.1 mM pyridoxal phosphate and 50 mM potassium phosphate buffer (pH 7.4) was added to the microbial cells collected from 2 ml of the microbial cell suspension by centrifugation (18,000×g, 5 minutes, 4°C). The mixture was reacted at 30°C for one hour. After the completion of the reaction, the ESI-MS analysis was performed using a supernatant obtained by centrifugation (18,000×g, 5 minutes, 4°C), and a molecular ion peak of α-propylserine was observed.

### <Example 34> Synthesis of α-butylserine

50 ml of the LB medium containing 100 mg/l of ampicillin in a 500 ml Sakaguchi flask was inoculated with JM109/pSFNRHMT-Y339S cultured overnight in the LB agar medium containing 100 mg/l ampicillin, and cultured at 37°C at 120 reciprocations/min for 17 hours. The microbial cells were collected from 10 ml of the cultured medium by centrifugation (8,000×g, 10 minutes), and then washed with 10 ml of 50 mM potassium phosphate buffer (pH 7.4) containing 0.1 mM pyridoxal phosphate. A microbial cell suspension was prepared using 10 ml of the same buffer. Then 100 µl of a solution composed of 10 mM formaldehyde, 30 mM L-norleucine, 0.1 mM pyridoxal phosphate and 50 mM potassium phosphate buffer (pH 7.4) was added to the microbial cells collected from 2 ml of the microbial cell suspension by centrifugation (18,000×g, 5 minutes, 4°C). The mixture was reacted at 30°C for one hour. After the completion of the reaction, the ESI-MS analysis was performed using a supernatant obtained by centrifugation (18,000xg, 5 minutes, 4°C), and a molecular ion peak of α-butylserine was observed.

### <Example 35> Synthesis of methylserine using cell-free extract solution of JM109/pSFNRHMT-Y339S-N19S

50 ml of the TB medium (12 g/l of trypton, 24 g/l of yeast extract, 2.3 g/l of potassium dihydrogen phosphate, 12.5 g/l of dipotassium hydrogen phosphate, and 4 g/l of glycerol) containing 100 mg/l of ampicillin in a 500 ml Sakaguchi flask was inoculated with JM109/pSFNRHMT-Y339S-N19S cultured overnight in the LB agar medium containing 100 mg/l of ampicillin, and cultured at 37°C at 120 reciprocations/min for 16 hours. The microbial cells were collected from 40 ml of the cultured medium by centrifugation (8,000xg, 10 minutes), and then washed with 40 ml of 50 mM potassium phosphate buffer (pH 7.4) containing 0.1 mM pyridoxal phosphate. A microbial cell suspension was prepared using 4 ml of the same buffer. The microbial cell suspension was sonicated to disrupt the microbial cells. A supernatant obtained by centrifugation (18,000×g, 5 minutes, 4°C) was used as a cell-free extract solution. 0.034 ml of the resulting cell-free extract solution was added to the reaction solution composed of 30 mM formaldehyde, 30 mM L-alanine, 0.1 mM pyridoxal phosphate and 50 mM potassium phosphate buffer (pH 7.4) to make a reaction mixture in a total amount of 0.1 ml. The reaction mixture was reacted at 30°C for one hour. The special grade formaldehyde solution [code number: 16223-55] supplied from Nacalai Tesque Inc. was used as formaldehyde. After the completion of the reaction, 100 µl of 4 mM copper sulfate was added to the reaction mixture, and centrifuged (18,000×g, 5 minutes, 4°C). Then, 100 µl of water was added to 100 µl of the resulting solution, which was then analyzed by HPLC using SumichiralOA-5000 (Sumika Chemical Analysis Service Ltd.) (Mobile phase: aqueous solution of 0.5 mM copper sulfate, column temperature: 30°C, flow rate: 1 ml/minute, detection: UV 230 nm). As a result, it was confirmed that 19.5 mM α-methyl-L-serine had been formed.

### INDUSTRIAL APPLICABILITY

The present invention is useful in industries involved in the methods for producing the amino acids. The present invention is expected to contribute to the production of the various serine derivatives and the optical active amino acids and to be used, for example, as the method for producing pharmaceutical intermediates.

### Sequence listing free text

SEQ ID NO:1 N-terminal amino acid sequence of 2-benzylserine hydroxymethyltransferase derived from *Rhodococcus sp.* (AJ110611 strain)
SEQ ID NO:2 Primer SEQA
SEQ ID NO:3 Primer SEQB
SEQ ID NO:4 Nucleic acid sequence (ORF) encoding 2-benzylserine hydroxymethyltransferase derived from *Rhodococcus sp.* (AJ110611 strain)
SEQ ID NO:5 Amino acid sequence of 2-benzylserine hydroxymethyltransferase derived from *Rhodococcus sp.* (AJ110611 strain)
SEQ ID NO:6 Primer S11F
SEQ ID NO:7 Primer S12R
SEQ ID NO:8 Primer Y339SF
SEQ ID NO:9 Primer Y339SR
SEQ ID NO:10 Primer Y339HF
SEQ ID NO:11 Primer Y339HR
SEQ ID NO:12 Primer Y339NF
SEQ ID NO:13 Primer Y339NR
SEQ ID NO:14 Primer N19SF
SEQ ID NO:15 Primer N19SR

## Claims

1. A method for producing a serine derivative, comprising reacting an α-amino acid represented by formula (I): 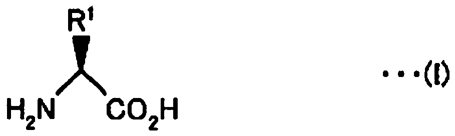 with an aldehyde represented by formula (II): 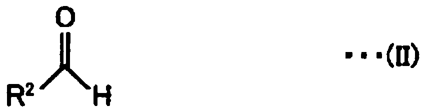 in the presence of an enzyme to form the serine derivative represented by formula (III): 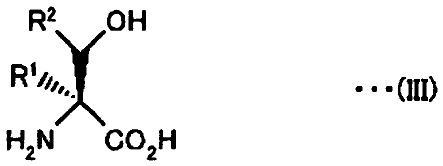 wherein R¹ of formula (I) is selected from the group consisting of an alkyl group having 1 to 7 carbon atoms, an aryl group having 6 to 14 carbon atoms, a cycloalkyl group having 3 to 10 carbon atoms, an aralkyl group having 7 to 19 carbon atoms, an alkoxyalkyl group having 2 to 11 carbon atoms, a group containing a hetero atom in the carbon skeleton thereof, and a group containing a carbon-carbon unsaturated bond in the carbon skeleton thereof, wherein these groups may be straight or branched, may have an alicyclic hydrocarbon structure and may further have a substituent,
wherein R² of formula (II) is selected from the group consisting of hydrogen, an alkyl group having 1 to 7 carbon atoms, an aryl group having 6 to 14 carbon atoms, a cycloalkyl group having 3 to 10 carbon atoms, an aralkyl group having 7 to 19 carbon atoms, an alkoxyalkyl group having 2 to 11 carbon atoms, a group containing a hetero atom in the carbon skeleton thereof, and a group containing a carbon-carbon unsaturated bond in the carbon skeleton thereof, wherein these groups may be straight or branched, may have an alicyclic hydrocarbon structure and may further have a substituent,
wherein R¹ of formula (III) is the same as R¹ of formula (I) and R² of formula (III) is the same as R² of formula (II);
wherein the enzyme is one or two or more proteins selected from the group consisting of the following (A) and (B):
(A) a protein having the amino acid sequence of SEQ ID NO:5, and
(B) a protein having an amino acid sequence comprising mutation of 1 to 100 amino acids which is selected from the group consisting of substitution, deletion, insertion and addition in the amino acid sequence of SEQ ID NO:5 and having an activity to catalyze the reaction to form the serine derivative represented by formula (III).

2. The method for producing the serine derivative according to claim 1, wherein the protein (B) is a protein comprising a sequence in which tyrosine located at position 339 is substituted with any one amino acid selected from the group consisting of serine, histidine and asparagine in the amino acid sequence of SEQ ID NO:5.

3. The method for producing the serine derivative according to claim 1 or 2, wherein the protein (B) is a protein comprising a sequence in which asparagine located at position 19 is substituted with serine in the amino acid sequence of SEQ ID NO:5.

4. The method for producing a serine derivative according to any one of claims 1 to 3,
wherein the protein is derived from a microorganism belonging to genus *Rhodococcus.*

5. The method for producing the serine derivative according to any one of claims 1 to 4, wherein the microorganism producing the enzyme, the α-amino acid represented by formula (I) and the aldehyde represented by formula (II) are mixed to form the serine derivative represented by formula (III).

6. The method for producing the serine derivative according to any one of claims 1 to 4, wherein a culture comprising the microorganism producing the enzyme, the α-amino acid represented by formula (I) and the aldehyde represented by formula (II) are mixed to form the serine derivative represented by formula (III).

7. The method for producing the serine derivative according to any one of claims 1 to 4, wherein a treated microbial cell product of the microorganism producing the enzyme, the α-amino acid represented by formula (I) and the aldehyde represented by formula (II) are mixed to form the serine derivative represented by formula (III).

8. The method for producing the serine derivative according to any one of claims 1 to 7, wherein the amino acid represented by formula (I) is one or two or more amino acids selected from the group consisting of phenylalanine, leucine, methionine, alanine, cysteine, tryptophan, isoleucine, cyclohexylalanine, 2-amino-n-butyric acid, 2-aminovaleric acid and 2-aminohexanoic acid.

9. The method for producing the serine derivative according to any one of claims 1 to 7, wherein the amino acid represented by formula (I) is α-phenylalanine and the serine derivative represented by formula (III) is α-benzylserine.

10. The method for producing the serine derivative according to any one of claims 1 to 7, wherein the amino acid represented by formula (I) is α-leucine and the serine derivative represented by formula (III) is α-isobutylserine.

11. The method for producing the serine derivative according to any one of claims 1 to 7, wherein the amino acid represented by formula (I) is α-methionine and the serine derivative represented by formula (III) is α-methylthioethylserine.

12. A protein selected from the group consisting of the following (A) and (B):
(A) a protein having the amino acid sequence of SEQ ID NO:5, and
(B) a protein having an amino acid sequence comprising mutation of 1 to 100 amino acids which is selected from the group consisting of substitution, deletion, insertion and addition in the amino acid sequence of SEQ ID NO:5,
wherein the protein has an activity to catalyze a reaction in which an α-amino acid represented by formula (I) : 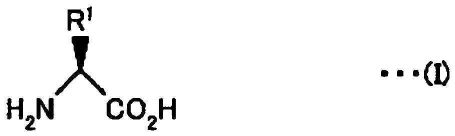 is reacted with an aldehyde represented by formula (II): 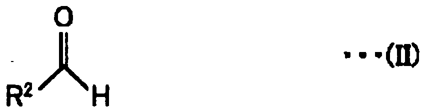 to form a serine derivative represented by formula (III): 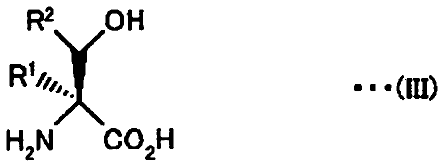 wherein R¹ of formula (I) is selected from the group consisting of an alkyl group having 1 to 7 carbon atoms, an aryl group having 6 to 14 carbon atoms, a cycloalkyl group having 3 to 10 carbon atoms, an aralkyl group having 7 to 19 carbon atoms, an alkoxyalkyl group having 2 to 11 carbon atoms, a group containing a hetero atom in the carbon skeleton thereof, and a group containing a carbon-carbon unsaturated bond in the carbon skeleton thereof, wherein these groups may be straight or branched, may have an alicyclic hydrocarbon structure and may further have a substituent,
wherein R² of formula (II) is selected from the group consisting of hydrogen, an alkyl group having 1 to 7 carbon atoms, an aryl group having 6 to 14 carbon atoms, a cycloalkyl group having 3 to 10 carbon atoms, an aralkyl group having 7 to 19 carbon atoms, an alkoxyalkyl group having 2 to 11 carbon atoms, a group containing a hetero atom in the carbon skeleton thereof, and a group containing a carbon-carbon unsaturated bond in the carbon skeleton thereof, wherein these groups may be straight or branched, may have an alicyclic hydrocarbon structure and may further have a substituent,
wherein R¹ of formula (III) is the same as R¹ of formula (I) and R² of formula (III) is the same as R² of formula (II).

13. The protein according to claim 12, which is derived from a microorganism belonging to genus Rhodococcus.

14. The protein according to claim 13, wherein the protein (B) comprises a sequence in which tyrosine located at position 339 is substituted with any one amino acid selected from the group consisting of serine, histidine and asparagine in the amino acid sequence of SEQ ID NO:5.

15. The protein according to claim 14, wherein the protein (B) comprises a sequence in which asparagine located at position 19 is further substituted with serine in the amino acid sequence of SEQ ID NO:5

16. A polynucleotide encoding the protein according to any one of claims 12 to 15.

17. A polynucleotide selected from the group consisting of the following (a) and (b):
(a) a polynucleotide having the nucleotide sequence of SEQ ID NO:4, and
(b) a polynucleotide that hybridizes with a polynucleotide having a nucleotide sequence complementary to the nucleotide sequence of SEQ ID NO:4 under a stringent condition and encodes a protein having an activity to catalyze a reaction in which an α-amino acid represented by formula (I): 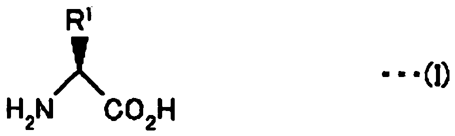 is reacted with an aldehyde represented by formula (II): 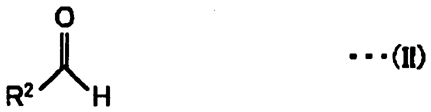 to form a serine derivative represented by formula (III): 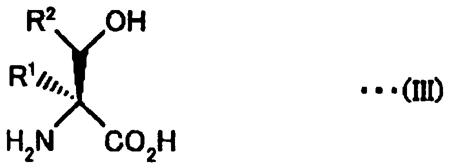 wherein R¹ of formula (I) is selected from the group consisting of an alkyl group having 1 to 7 carbon atoms, an aryl group having 6 to 14 carbon atoms, a cycloalkyl group having 3 to 10 carbon atoms, an aralkyl group having 7 to 19 carbon atoms, an alkoxyalkyl group having 2 to 11 carbon atoms, a group containing a hetero atom in the carbon skeleton thereof, and a group containing a carbon-carbon unsaturated bond in the carbon skeleton thereof, wherein these groups may be straight or branched, may have an alicyclic hydrocarbon structure and may further have a substituent,
wherein R² of formula (II) is selected from the group consisting of hydrogen, an alkyl group having 1 to 7 carbon atoms, an aryl group having 6 to 14 carbon atoms, a cycloalkyl group having 3 to 10 carbon atoms, an aralkyl group having 7 to 19 carbon atoms, an alkoxyalkyl group having 2 to 11 carbon atoms, a group containing a hetero atom in the carbon skeleton thereof, and a group containing a carbon-carbon unsaturated bond in the carbon skeleton thereof, wherein these groups may be straight or branched, may have an alicyclic hydrocarbon structure and may further have a substituent,
wherein R¹ of formula (III) is the same as R¹ of formula (I) and R² of formula (III) is the same as R² of formula (II).

18. A cell transformed with the polynucleotide according to claim 16 or 17.

19. The cell according to claim 18, wherein the cell is a transformed *Escherichia coli* cell as a host.

## Patentansprüche

1. Verfahren zur Herstellung eines Serin-Derivats, umfassend das zur Reaktion Bringen einer α-Aminosäure, die durch die Formel (I) dargestellt ist: 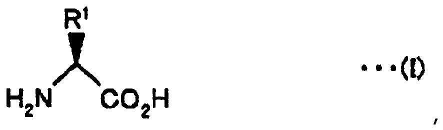 mit einem Aldehyd, der durch die Formel (II) dargestellt ist: 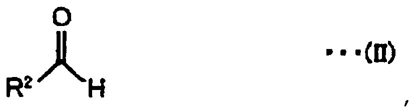 in der Gegenwart eines Enzyms, um das Serin-Derivat zu bilden, das durch die Formel (III) dargestellt ist: 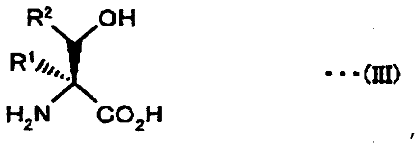 wobei R¹ der Formel (I) ausgewählt ist aus der Gruppe bestehend aus einer Alkylgruppe mit 1 bis 7 Kohlenstoffatomen, einer Arylgruppe mit 6 bis 14 Kohlenstoffatomen, einer Cycloalkylgruppe mit 3 bis 10 Kohlenstoffatomen, einer Aralkylgruppe mit 7 bis 19 Kohlenstoffatomen, einer Alkoxyalkylgruppe mit 2 bis 11 Kohlenstoffatomen, einer Gruppe, die ein Heteroatom in ihrem Kohlenstoffskelett enthält, und einer Gruppe, die eine ungesättigte Kohlenstoff-Kohlenstoff-Bindung in ihrem Kohlenstoffskelett enthält, wobei diese Gruppen gerade oder verzweigt sein können, eine alicyclische Kohlenwasserstoffstruktur haben können und ferner einen Substituenten haben können,
wobei R² der Formel (II) ausgewählt ist aus der Gruppe bestehend aus Wasserstoff, einer Alkylgruppe mit 1 bis 7 Kohlenstoffatomen, einer Arylgruppe mit 6 bis 14 Kohlenstoffatomen, einer Cycloalkylgruppe mit 3 bis 10 Kohlenstoffatomen, einer Aralkylgruppe mit 7 bis 19 Kohlenstoffatomen, einer Alkoxyalkylgruppe mit 2 bis 11 Kohlenstoffatomen, einer Gruppe, die ein Heteroatom in ihrem Kohlenstoffskelett enthält, und einer Gruppe, die eine ungesättigte Kohlenstoff-Kohlenstoff-Bindung in ihrem Kohlenstoffskelett enthält, wobei diese Gruppen gerade oder verzweigt sein können, eine alicyclische Kohlenwasserstoff-Struktur haben können und ferner einen Substituenten haben können,
wobei R¹ der Formel (III) gleich R¹ der Formel (I) ist, und R² der Formel (III) gleich R² der Formel (II) ist;
wobei das Enzym ein oder zwei oder mehr Proteine ist, die aus der Gruppe bestehend aus den folgenden (A) und (B) ausgewählt sind:
(A) ein Protein mit der Aminosäuresequenz von SEQ ID NO: 5, und
(B) ein Protein mit einer Aminosäuresequenz, die Mutation von 1 bis 100 Aminosäuren umfasst, die ausgewählt ist aus der Gruppe bestehend aus Substitution, Deletion, Insertion und Addition in der Aminosäuresequenz von SEQ ID NO: 5, und mit einer Aktivität, um die Reaktion zur Bildung des Serin-Derivats zu katalysieren, das durch die Formel (III) dargestellt ist.

2. Verfahren zur Herstellung des Serin-Derivats gemäß Anspruch 1, wobei das Protein (B) ein Protein ist, das eine Sequenz umfasst, in der das sich an Position 339 befindende Tyrosin in der Aminosäuresequenz von SEQ ID NO: 5 mit einer beliebigen Aminosäure substituiert ist, die ausgewählt ist aus der Gruppe bestehend aus Serin, Histidin und Asparagin.

3. Verfahren zur Herstellung des Serin-Derivats gemäß Anspruch 1 oder 2, wobei das Protein (B) ein Protein ist, das eine Sequenz umfasst, in der das sich an Position 19 befindende Asparagin in der Aminosäuresequenz von SEQ ID NO: 5 mit Serin substituiert ist.

4. Verfahren zur Herstellung eines Serin-Derivats gemäß mindestens einem der Ansprüche 1 bis 3, wobei das Protein aus einem Mikroorganismus stammt, der zu der Gattung Rhodococcus gehört.

5. Verfahren zur Herstellung des Serin-Derivats gemäß mindestens einem der Ansprüche 1 bis 4, wobei der Mikroorganismus, der das Enzym herstellt, die durch die Formel (I) dargestellte α-Aminosäure und der durch die Formel (II) dargestellte Aldehyd gemischt werden, um das Serin-Derivat, das durch die Formel (III) dargestellt ist, zu bilden.

6. Verfahren zur Herstellung des Serin-Derivats gemäß mindestens einem der Ansprüche 1 bis 4, wobei eine Kultur, die den Mikroorganismus umfasst, der das Enzym herstellt, die durch die Formel (I) dargestellte α-Aminosäure und der durch die Formel (II) dargestellte Aldehyd gemischt werden, um das Serin-Derivat zu bilden, das durch die Formel (III) dargestellt ist.

7. Verfahren zur Herstellung des Serin-Derivats gemäß mindestens einem der Ansprüche 1 bis 4, wobei ein Produkt einer behandelten mikrobiellen Zelle des Mikroorganismus, der das Enzym herstellt, die durch die Formel (I) dargestellte α-Aminosäure und der durch die Formel (II) dargestellte Aldehyd gemischt werden, um das Serin-Derivat zu bilden, das durch die Formel (III) dargestellt ist.

8. Verfahren zur Herstellung des Serin-Derivats gemäß mindestens einem der Ansprüche 1 bis 7, wobei die durch die Formel (I) dargestellte Aminosäure eine oder zwei oder mehr Aminosäuren ist, die ausgewählt sind aus der Gruppe bestehend aus Phenylalanin, Leucin, Methionin, Alanin, Cystein, Tryptophan, Isoleucin, Cyclohexylalanin, 2-Amino-n-buttersäure, 2-Aminopentansäure und 2-Aminohexansäure.

9. Verfahren zur Herstellung des Serin-Derivats gemäß mindestens einem der Ansprüche 1 bis 7, wobei die durch die Formel (I) dargestellte Aminosäure α-Phenylalanin ist und das durch die Formel (III) dargestellte Serin-Derivat α-Benzylserin ist.

10. Verfahren zur Herstellung des Serin-Derivats gemäß mindestens einem der Ansprüche 1 bis 7, wobei die durch die Formel (I) dargestellte Aminosäure α-Leucin ist und das durch die Formel (III) dargestellte Serin-Derivat α-Isobutylserin ist.

11. Verfahren zur Herstellung des Serin-Derivats gemäß mindestens einem der Ansprüche 1 bis 7, wobei die durch die Formel (I) dargestellte Aminosäure α-Methionin ist und das durch die Formel (III) dargestellte Serin-Derivat α-Methylthioethylserin ist.

12. Protein, ausgewählt aus der Gruppe bestehend aus den folgenden (A) und (B):
(A) ein Protein mit der Aminosäuresequenz von SEQ ID NO: 5, und
(B) ein Protein mit einer Aminosäuresequenz, die Mutation von 1 bis 100 Aminosäuren umfasst, die ausgewählt ist aus der Gruppe bestehend aus Substitution, Deletion, Insertion und Addition in der Aminosäuresequenz von SEQ ID NO: 5,
wobei das Protein eine Aktivität hat, eine Reaktion zu katalysieren, in der eine α-Aminosäure, die durch die Formel (I) dargestellt ist: 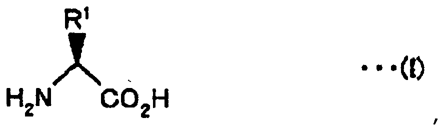 mit einem Aldehyd, der durch die Formel (II) dargestellt ist: 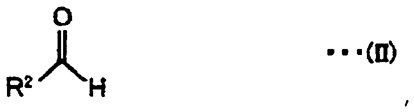 zur Reaktion gebracht wird,
um ein Serin-Derivat zu bilden, das durch die Formel (III) dargestellt ist: 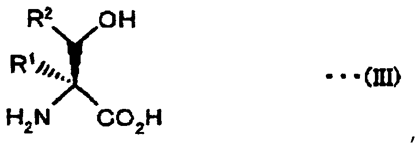 wobei R¹ der Formel (I) ausgewählt ist aus der Gruppe bestehend aus einer Alkylgruppe mit 1 bis 7 Kohlenstoffatomen, einer Arylgruppe mit 6 bis 14 Kohlenstoffatomen, einer Cycloalkylgruppe mit 3 bis 10 Kohlenstoffatomen, einer Aralkylgruppe mit 7 bis 19 Kohlenstoffatomen, einer Alkoxyalkylgruppe mit 2 bis 11 Kohlenstoffatomen, einer Gruppe, die ein Heteroatom in ihrem Kohlenstoffskelett enthält, und einer Gruppe, die eine ungesättigte Kohlenstoff-Kohlenstoff-Bindung in ihrem Kohlenstoffskelett enthält, wobei diese Gruppen gerade oder verzweigt sein können, eine alicyclische Kohlenwasserstoff-Struktur haben können und ferner einen Substituenten haben können,
wobei R² der Formel (II) ausgewählt ist aus der Gruppe bestehend aus Wasserstoff, einer Alkylgruppe mit 1 bis 7 Kohlenstoffatomen, einer Arylgruppe mit 6 bis 14 Kohlenstoffatomen, einer Cycloalkylgruppe mit 3 bis 10 Kohlenstoffatomen, einer Aralkylgruppe mit 7 bis 19 Kohlenstoffatomen, einer Alkoxyalkylgruppe mit 2 bis 11 Kohlenstoffatomen, einer Gruppe, die ein Heteroatom in ihrem Kohlenstoffskelett enthält, und einer Gruppe, die eine ungesättigte Kohlenstoff-Kohlenstoff-Bindung in ihrem Kohlenstoffskelett enthält, wobei diese Gruppen gerade oder verzweigt sein können, eine alicyclische Kohlenwasserstoff-Struktur haben können und ferner einen Substituenten haben können, wobei R¹ der Formel (III) gleich R¹ der Formel (I) ist, und R² der Formel (III) gleich R² der Formel (II) ist.

13. Protein gemäß Anspruch 12, das aus einem Mikroorganismus stammt, der zur Gattung Rhodococcus gehört.

14. Protein gemäß Anspruch 13, wobei das Protein (B) eine Sequenz umfasst, in der das sich an Position 339 befindende Tyrosin in der Aminosäuresequenz von SEQ ID NO: 5 mit einer beliebigen Aminosäure substituiert ist, die ausgewählt ist aus der Gruppe bestehend aus Serin, Histidin und Asparagin.

15. Protein gemäß Anspruch 14, wobei das Protein (B) eine Sequenz umfasst, in der das sich an Position 19 befindende Asparagin der Aminosäuresequenz von SEQ ID NO: 5 ferner mit Serin substituiert ist.

16. Polynukleotid, das das Protein gemäß mindestens einem der Ansprüche 12 bis 15 kodiert.

17. Polynukleotid, ausgewählt aus der Gruppe bestehend aus den folgenden (a) und (b):
(a) ein Polynukleotid mit der Nukleotidsequenz von SEQ ID NO: 4, und
(b) ein Polynukleotid, das mit einem Polynukleotid mit einer Nukleotidsequenz, die zu der Nukleotidsequenz von SEQ ID NO: 4 komplementär ist, unter einer stringenten Bedingung hybridisiert, und das ein Protein mit einer Aktivität kodiert, eine Reaktion zu katalysieren, in der eine α-Aminosäure, die durch die Formel (I) dargestellt ist: 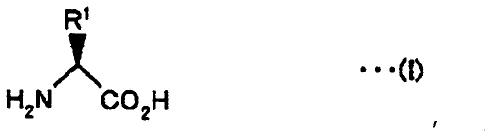 mit einem Aldehyd zur Reaktion gebracht wird, der durch die Formel (II) dargestellt ist: 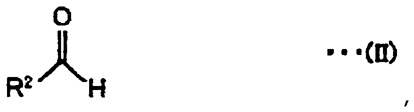 um ein Serin-Derivat zu bilden, das durch die Formel (III) dargestellt ist: 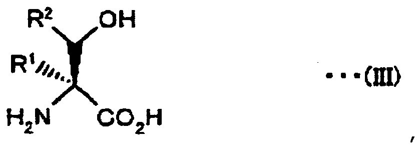 wobei R¹ der Formel (I) ausgewählt ist aus der Gruppe bestehend aus einer Alkylgruppe mit 1 bis 7 Kohlenstoffatomen, einer Arylgruppe mit 6 bis 14 Kohlenstoffatomen, einer Cycloalkylgruppe mit 3 bis 10 Kohlenstoffatomen, einer Aralkylgruppe mit 7 bis 19 Kohlenstoffatomen, einer Alkoxyalkylgruppe mit 2 bis 11 Kohlenstoffatomen, einer Gruppe, die ein Heteroatom in ihrem Kohlenstoffskelett enthält, und einer Gruppe, die eine ungesättigte Kohlenstoff-Kohlenstoff-Bindung in ihrem Kohlenstoffskelett enthält, wobei diese Gruppen gerade oder verzweigt sein können, eine alicyclische Kohlenwasserstoff-Struktur haben können und ferner einen Substituenten haben können,
wobei R² der Formel (II) ausgewählt ist aus der Gruppe bestehend aus Wasserstoff, einer Alkylgruppe mit 1 bis 7 Kohlenstoffatomen, einer Arylgruppe mit 6 bis 14 Kohlenstoffatomen, einer Cycloalkylgruppe mit 3 bis 10 Kohlenstoffatomen, einer Aralkylgruppe mit 7 bis 19 Kohlenstoffatomen, einer Alkoxyalkylgruppe mit 2 bis 11 Kohlenstoffatomen, einer Gruppe, die ein Heteroatom in ihrem Kohlenstoffskelett enthält, und einer Gruppe, die eine ungesättigte Kohlenstoff-Kohlenstoff-Bindung in ihrem Kohlenstoffskelett enthält, wobei diese Gruppen gerade oder verzweigt sein können, eine alicyclische Kohlenwasserstoff-Struktur haben können und ferner einen Substituenten haben können,
wobei R¹ der Formel (III) gleich R¹ der Formel (I) ist, und R² der Formel (III) gleich R² der Formel (II) ist.

18. Zelle, die mit dem Polynukleotid gemäß Anspruch 16 oder 17 transformiert ist.

19. Zelle gemäß Anspruch 18, wobei die Zelle eine transformierte Escherichia coli-Zelle als ein Wirt ist.

## Revendications

1. Procédé de production d'un dérivé de sérine, comprenant la réaction d'un acide α-aminé représenté par la formule (I) : 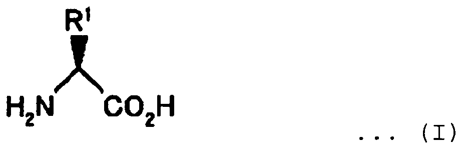 avec un aldéhyde représenté par formule (II) : 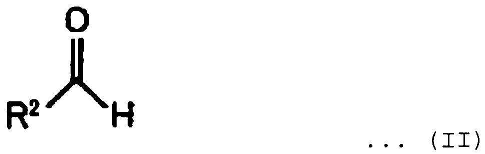 en présence d'une enzyme pour former le dérivé de sérine représenté par la formule (III) : 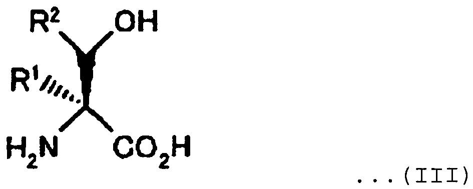 dans lequel R¹ de la formule (I) est sélectionné à partir du groupe constitué par un groupe alkyle ayant de 1 à 7 atomes de carbone, un groupe aryle ayant de 6 à 14 atomes de carbone, un groupe cycloalkyle ayant de 3 à 10 atomes de carbone, un groupe aralkyle ayant de 7 à 19 atomes de carbone, un groupe alcoxyalkyle ayant de 2 à 11 atomes de carbone, un groupe contenant un hétéroatome dans le squelette de carbone de ce dernier, et un groupe contenant une liaison carbone-carbone non saturée dans le squelette de carbone de ce dernier, dans lesquels ces groupes peuvent être linéaires ou ramifiés, peuvent avoir une structure d'hydrocarbure alicyclique et peuvent en outre avoir un substituant,
dans lequel R² de la formule (II) est sélectionné à partir du groupe constitué par l'hydrogène, un groupe alkyle ayant de 1 à 7 atomes de carbone, un groupe aryle ayant de 6 à 14 atomes de carbone, un groupe cycloalkyle ayant de 3 à 10 atomes de carbone, un groupe aralkyle ayant de 7 à 19 atomes de carbone, un groupe alcoxyalkyle ayant de 2 à 11 atomes de carbone, un groupe contenant un hétéroatome dans le squelette de carbone de ce dernier, et un groupe contenant une liaison carbone-carbone non saturée dans le squelette de carbone de ce dernier, dans lesquels ces groupes peuvent être linéaires ou ramifiés, peuvent avoir une structure d'hydrocarbure alicyclique et peuvent en outre avoir un substituant,
dans lequel R¹ de la formule (III) est le même que R¹ de la formule (I) et R² de la formule (III) est le même que R² de la formule (II) ;
dans lequel l'enzyme est une ou deux ou plusieurs protéines sélectionnées à partir du groupe constitué par les (A) et (B) suivants :
(A) une protéine ayant la séquence d'acides aminés de SEQ ID n° 5, et
(B) une protéine ayant une séquence d'acides aminés comprenant une mutation de 1 à 100 acides aminés qui est sélectionnée à parti du groupe consistant en substitution, délétion, insertion et addition dans la séquence d'acides aminés de SEQ ID n° 5 et ayant une activité pour catalyser la réaction pour former le dérivé de sérine représenté par la formule (III).

2. Procédé de production du dérivé de sérine selon la revendication 1, dans lequel la protéine (B) est une protéine comprenant une séquence dans laquelle de la tyrosine située à la position 339 est remplacée par n'importe quel acide aminé sélectionné à partir du groupe constitué par la sérine, l'histidine et l'asparagine dans la séquence d'acides aminés de SEQ ID n° 5.

3. Procédé de production du dérivé de sérine selon la revendication 1 ou 2, dans lequel la protéine (B) est une protéine comprenant une séquence dans laquelle de l'asparagine située à la position 19 est remplacée par de la sérine dans la séquence d'acides aminés de SEQ ID n° 5.

4. Procédé de production d'un dérivé de sérine selon l'une quelconque des revendications 1 à 3,
dans lequel la protéine est dérivée d'un micro-organisme appartenant au genre *Rhodococcus.*

5. Procédé de production du dérivé de sérine selon l'une quelconque des revendications 1 à 4, dans lequel le micro-organisme produisant l'enzyme, l'acide α-aminé représenté par la formule (I) et l'aldéhyde représenté par la formule (II) sont mélangés pour former le dérivé de sérine représenté par la formule (III).

6. Procédé de production du dérivé de sérine selon l'une quelconque des revendications 1 à 4, dans lequel une culture comprenant le micro-organisme produisant l'enzyme, l'acide α-aminé représenté par la formule (I) et l'aldéhyde représenté par la formule (II) sont mélangés pour former le dérivé de sérine représenté par la formule (III).

7. Procédé de production du dérivé de sérine selon l'une quelconque des revendications 1 à 4, dans lequel un produit de cellule microbienne traitée du micro-organisme produisant l'enzyme, l'acide α-aminé représenté par la formule (I) et l'aldéhyde représenté par la formule (II) sont mélangés pour former le dérivé de sérine représenté par la formule (III).

8. Procédé de production du dérivé de sérine selon l'une quelconque des revendications 1 à 7, dans lequel l'acide aminé représenté par la formule (I) est un ou deux ou plusieurs acides aminés sélectionnés à partir du groupe constitué par la phénylalanine, la leucine, la méthionine, l'alanine, la cystéine, le tryptophane, l'isoleucine, la cyclohexylalanine, l'acide 2-amino-n-butyrique, l'acide 2-aminovalérique et l'acide 2-aminohexanoïque.

9. Procédé de production du dérivé de sérine selon l'une quelconque des revendications 1 à 7, dans lequel l'acide aminé représenté par la formule (I) est une α-phénylalanine et le dérivé de sérine représenté par la formule (III) est une α-benzylsérine.

10. Procédé de production du dérivé de sérine selon l'une quelconque des revendications 1 à 7, dans lequel l'acide aminé représenté par la formule (I) est une α-leucine et le dérivé de sérine représenté par la formule (III) est une α-isobutylsérine.

11. Procédé de production du dérivé de sérine selon l'une quelconque des revendications 1 à 7, dans lequel l'acide aminé représenté par la formule (I) est une α-méthionine et le dérivé de sérine représenté par la formule (III) est une α-méthylthioéthylsérine.

12. Protéine sélectionnée à partir du groupe constitué par les (A) et (B) suivants :
(A) une protéine ayant la séquence d'acides aminés de SEQ ID n° 5, et
(B) une protéine ayant une séquence d'acides aminés comprenant une mutation de 1 à 100 acides aminés qui est sélectionnée à partir du groupe consistant en substitution, délétion, insertion et addition dans la séquence d'acides aminés de SEQ ID n° 5,
dans laquelle la protéine a une activité pour catalyser une réaction dans laquelle un acide α-aminé représenté par la formule (I) : 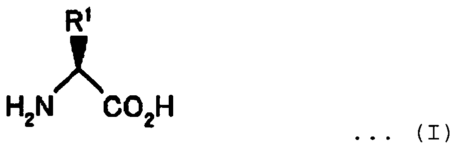 réagit avec un aldéhyde représenté par la formule (II) : 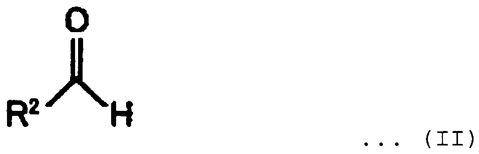 pour former un dérivé de sérine représenté par la formule (III) : 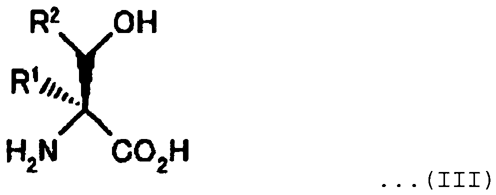 dans laquelle R¹ de la formule (I) est sélectionné à partir du groupe constitué par un groupe alkyle ayant de 1 à 7 atomes de carbone, un groupe aryle ayant de 6 à 14 atomes de carbone, un groupe cycloalkyle ayant de 3 à 10 atomes de carbone, un groupe aralkyle ayant de 7 à 19 atomes de carbone, un groupe alcoxyalkyle ayant de 2 à 11 atomes de carbone, un groupe contenant un hétéroatome dans le squelette de carbone de ce dernier, et un groupe contenant une liaison carbone-carbone non saturée dans le squelette de carbone de ce dernier, dans lesquels ces groupes peuvent être linéaires ou ramifiés, peuvent avoir une structure d'hydrocarbure alicyclique et peuvent en outre avoir un substituant,
dans laquelle R² de la formule (II) est sélectionné à partir du groupe constitué par l'hydrogène, un groupe alkyle ayant de 1 à 7 atomes de carbone, un groupe aryle ayant de 6 à 14 atomes de carbone, un groupe cycloalkyle ayant de 3 à 10 atomes de carbone, un groupe aralkyle ayant de 7 à 19 atomes de carbone, un groupe alcoxyalkyle ayant de 2 à 11 atomes de carbone, un groupe contenant un hétéroatome dans le squelette carbonique de ce dernier, et un groupe contenant une liaison carbone-carbone non saturée dans le squelette de carbone de ce dernier, dans lesquels ces groupes peuvent être linéaires ou ramifiés, peuvent avoir une structure d'hydrocarbure alicyclique et peuvent en outre avoir un substituant,
dans laquelle R¹ de la formule (III) est le même que R¹ de la formule (I) et R² de la formule (III) est le même que R² de la formule (II).

13. Protéine selon la revendication 12, qui est dérivée d'un micro-organisme appartenant au genre *Rhodococcus.*

14. Protéine selon la revendication 13, dans laquelle la protéine (B) comprend une séquence dans laquelle de la tyrosine située à la position 339 est remplacée par n'importe quel acide aminé sélectionné à partir du groupe constitué par la sérine, l'histidine et l'asparagine dans la séquence d'acides aminés de SEQ ID n° 5.

15. Protéine selon la revendication 14, dans lequel la protéine (B) comprend une séquence dans laquelle de l'asparagine située à la position 19 est en outre remplacée par de la sérine dans la séquence d'acides aminés de SEQ ID n° 5.

16. Polynucléotide codant la protéine selon l'une quelconque des revendications 12 à 15.

17. Polynucléotide sélectionné à partir du groupe constitué par les (a) et (b) suivants :
(a) un polynucléotide ayant la séquence de nucléotides de SEQ ID n° 4, et
(b) un polynucléotide qui s'hybride avec un polynucléotide ayant une séquence de nucléotides complémentaire à la séquence de nucléotides de SEQ ID n° 4 sous une condition rigoureuse et qui code une protéine ayant une activité pour catalyser une réaction dans laquelle un acide α-aminé représenté par la formule (I) : 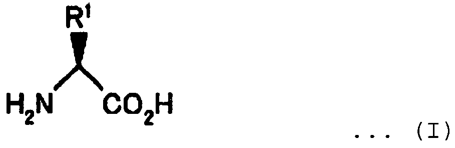 réagit avec un aldéhyde représenté par la formule (II) : 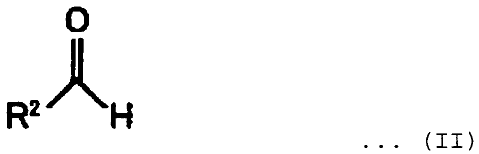 pour former un dérivé de sérine représenté par la formule (III) : 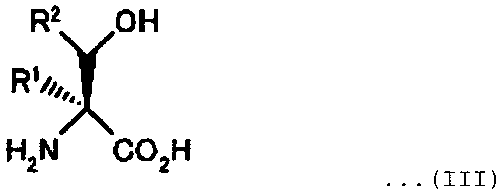 dans lequel R¹ de la formule (I) est sélectionné à partir du groupe constitué par un groupe alkyle ayant de 1 à 7 atomes de carbone, un groupe aryle ayant de 6 à 14 atomes de carbone, un groupe cycloalkyle ayant de 3 à 10 atomes de carbone, un groupe aralkyle ayant de 7 à 19 atomes de carbone, un groupe alcoxyalkyle ayant de 2 à 11 atomes de carbone, un groupe contenant un hétéroatome dans le squelette de carbone de ce dernier, et un groupe contenant une liaison carbone-carbone non saturée dans le squelette de carbone de ce dernier, dans lesquels ces groupes peuvent être linéaires ou ramifiés, peuvent avoir une structure d'hydrocarbure alicyclique et peuvent en outre avoir un substituant,
dans lequel R² de la formule (II) est sélectionné à partir du groupe constitué par l'hydrogène, un groupe alkyle ayant de 1 à 7 atomes de carbone, un groupe aryle ayant de 6 à 14 atomes de carbone, un groupe cycloalkyle ayant de 3 à 10 atomes de carbone, un groupe aralkyle ayant de 7 à 19 atomes de carbone, un groupe alcoxyalkyle ayant de 2 à 11 atomes de carbone, un groupe contenant un hétéroatome dans le squelette de carbone de ce dernier, et un groupe contenant une liaison carbone-carbone non saturée dans le squelette de carbone de ce dernier, dans lesquels ces groupes peuvent être linéaires ou ramifiés, peuvent avoir une structure d'hydrocarbure alicyclique et peuvent en outre avoir un substituant,
dans lequel R¹ de la formule (III) est le même que R¹ de la formule (I) et R² de la formule (III) est le même que R² de la formule (II).

18. Cellule transformée avec le polynucléotide selon la revendication 16 ou 17.

19. Cellule selon la revendication 18, dans laquelle la cellule est une cellule *Escherichia coli* transformée en tant qu'hôte.
